# EUROPEAN PATENT SPECIFICATION

(11) **EP 1 252 306 B1**
(45) Date of publication and mention of the grant of the patent: **22.07.2009**
(21) Application number: 01902616.0
(22) Date of filing: 31.01.2001
(51) Int. Cl.: C12N 15/11, C12N 15/63, C12N 15/867, A61K 31/7088, C12N 5/10

(54) **SELECTIVE KILLING OF CELLS BY ACTIVATION OF DOUBLE-STRANDED RNA DEPENDENT PROTEIN KINASE-PKR**
SELEKTIV-ABTÖTEN VON ZELLEN DURCH AKTIVIERUNG DER DOPPELSTRÄNGIGES RNS ABHÄNGIGEN PROTEINKINASE - PKR
CELLULES TUEES DE MANIERE SELECTIVE PAR ACTIVATION D'UN ARN DOUBLE BRIN DEPENDANT DE LA PROTEINE KINASE PKR

(30) Priority: 31.01.2000 US 179361 P; 22.12.2000 US 258010 P
(43) Date of publication of application: 30.10.2002
(73) Proprietor: YISSUM RESEARCH DEVELOPMENT COMPANY OF THE HEBREW UNIVERSITY OF JERUSALEM, 91390 Jerusalem (IL)
(72) Inventor: SHIR, Alexei, 24410 Acre (IL); LEVITZKI, Alexander, 94713 Jerusalem (IL)
(74) Representative: Machtalère, Georges
(86) International application number: PCT/IL2001/000094
(87) International publication number: WO 2001/057205

(56) References cited:
- WO-A-97/17456
- WO-A-98/54315
- HE YUKAI ET AL: "Inhibition of human squamous cell carcinoma growth in vivo by epidermal growth factor receptor antisense RNA transcribed from the U6 promoter." JOURNAL OF THE NATIONAL CANCER INSTITUTE (BETHESDA), vol. 90, no. 14, 15 July 1998 (1998-07-15), pages 1080-1087, XP001009921 ISSN: 0027-8874 cited in the application
- BEN-ASOULI Y ET AL: "Human interferon-gamma mRNA regulates its own translation through local activation of the protein kinase PKR." JOURNAL OF INTERFERON AND CYTOKINE RESEARCH, vol. 19, no. SUPPL. 1, September 1999 (1999-09), page S101 XP001009918 ISSN: 1079-9907 & Meeting of the International Society for Interferon and Cytokine Research with the participation of the European Cytokine Society;Paris, France; September 5-9, 1999
- DEGLON NICOLE ET AL: "Self-inactivating lentiviral vectors with enhanced transgene expression as potential gene transfer system in Parkinson's disease." HUMAN GENE THERAPY., vol. 11, no. 1, 1 January 2000 (2000-01-01), pages 179-190, XP001009919 ISSN: 1043-0342 cited in the application
- MANCHE LISA ET AL: "Interaction between double-stranded RNA regulators and the protein kinase DAI." MOLECULAR AND CELLULAR BIOLOGY, vol. 12, no. 11, 1992, pages 5238-5248, XP001009924 ISSN: 0270-7306 cited in the application
- JAGUS ROSEMARY ET AL: "PKR, apoptosis and cancer." INTERNATIONAL JOURNAL OF BIOCHEMISTRY & CELL BIOLOGY, vol. 31, no. 1, January 1999 (1999-01), pages 123-138, XP001009999 ISSN: 1357-2725

### SEQUENCE LISTING

<110> Shir, Alexei
   Levitzki, Alexander
<120> SELECTIVE KILLING OF CELLS BY ACTIVATION
   OF DOUBLE-STRANDED RNA DEPENDENT PROTEIN KINASE-PKR
<130> 1822/108
<160> 21
<170> FastSEQ for Windows Version 4.0
<210> 1
   <211> 36
   <212> RNA
   <213> Homo sapiens
<400> 1
   guuuuccuaa gacucuucca cucuauucuu gacuca 36
<210> 2
   <211> 36
   <212> DNA
   <213> Homo sapiens
<400> 2
   caaaagcatt ctgagaaggt gagataagaa ctgagt 36
<210> 3
   <211> 40
   <212> RNA
   <213> Homo sapiens
<400> 3
   acuuaaaaua aaaaaaacuu ccccugaauc acuacagaca 40
<210> 4
   <211> 40
   <212> DNA
   <213> Homo sapiens
<400> 4
   tgaattttat tttttttgaa ggggtcttag tgatgtctga 40
<210> 5
   <211> 40
   <212> RNA
   <213> Homo sapiens
<400> 5
   cuguuaacua cuggaccuuc acauggcggc cuucgugguc 40
<210> 6
   <211> 40
   <212> DNA
   <213> Homo sapiens
<400> 6
   gacaattgat gacctggaag tgtaccgccg gaagcaccag 40
<210> 7
   <211> 40
   <212> RNA
   <213> Homo sapiens
<400> 7
   ccgagaccuc cuuuucuuuc cauuaauaca ccacugucua 40
<210> 8
   <211> 40
   <212> DNA
   <213> Homo sapiens
<400> 8
   ggctctggag gaaaagaaag gtaattatgt ggtgacagat 40
<210> 9
   <211> 39
   <212> DNA
   <213> Homo sapiens
<400> 9
   tctgtcacca cataattacc tttcgtttcc tccagagcc 39
<210> 10
   <211> 39
   <212> DNA
   <213> Homo sapiens
<400> 10
   gctctggagg aaaagaaagg taattatgtg gtgacagat 39
<210> 11
   <211> 47
   <212> DNA
   <213> Homo sapiens
<400> 11
   tcgaggtgct tccggcggta cactactaag tgctgtccac taatgca 47
<210> 12
   <211> 47
   <212> DNA
   <213> Homo sapiens
<400> 12
   tcgagtagtg gacagcactt agtagtgtac cgccggaagc acatgca 47
<210> 13
   <211> 85
   <212> DNA
   <213> Homo sapiens
<400> 13
   tcgaggacgt ccataccgta tcttcatccc attcgcacac agggggtaac ggggcgccgg 60
   gtaagcacca ctgcatttca atgca 85
<210> 14
   <211> 85
   <212> RNA
   <213> Homo sapiens
<400> 14
   tcgagtgaaa tgcagtggtg cttacccggc gccccgttac cccctgtgtg cgaatgggat 60
   gaagatacgg tatggacgtc atgca 85
<210> 15
   <211> 24
   <212> DNA
   <213> Artificial Sequence
<220>
   <223> primer_bind
<400> 15
   acctgaggag acggtgacca gggt 24
<210> 16
   <211> 24
   <212> DNA
   <213> artifical sequence
<220>
   <221> primer_bind
   <222> (1)...(24)
<400> 16
   agttatggcc tatacactat ttgt 24
<210> 17
   <211> 20
   <212> DNA
   <213> Artifical sequence
<220>
   <221> primer_bind
   <222> (1)...(24)
<400> 17
   ttgtgagcaa aggtgatcgt 20
<210> 18
   <211> 19
   <212> DNA
   <213> artifical sequence
<220>
   <221> primer_bind
   <222> (1)...(20)
<400> 18
   cagggtccct tggccccag 19
<210> 19
   <211> 50
   <212> DNA
   <213> Homo sapiens
<400> 19
   ctccttccgc gggggctttc tcatggctgt ccttcagggt cttcctgaaa 50
<210> 20
   <211> 50
   <212> DNA
   <213> Homo sapiens
<400> 20
   tgcagtggtg cttacccggc gccccgttac cccctgtgtg cgaatgggat 50
<210> 21
   <211> 33
   <212> DNA
   <213> Homo sapiens
<220>
   <221> misc_feature
   <222> 9
   <223> n=a, t, c, or g
<400> 21
   gaaaatacng tatggacgtc tggggccaag gga 33

## Description

The present invention relates to a method of in vitro killing or inhibiting growth of a target cell according to claims 1 and 2, a method of in vitro killing, according to claims 3 and 4, the use of nucleic acid as a pharmaceutical according to claim 12, the use of a nucleic acid in the manufacture of a medicament for treating a disorder according to claims 12, 13 and 14, a pharmaceutical composition according to claims 21, 22 and 23, a composition comprising an antisense RNA according to claim 28, a nucleic acid according to claim 29, a vector according to claim 30, a cell according to claim 33.

Compositions and methods are provided for activating double stranded RNA dependent protein kinase that includes an anti-sense RNA with sequence homology with a locus in a target cell characterized by a mutation, that is absent in non-target cells.

Double stranded RNA dependent protein kinase (PKR) is a member of a family of kinases that phosphorylates the alpha subunit of protein synthesis initiation factor, eIF-2 (eIF-2α) and plays a role in the translational down regulation of gene expression (Clemens et al. Mol. Biol. Rep. 1994; vol. 19: 210-10). Activation of PKR involves two molecules binding in landem to double stranded RNA and then phosphorylating each other in an intramolecular event. (Wu et al. 1997, J. Biol. Chem 272:1291-1296). PKR has been implicated in processes that rely on apoptosis as control mechanisms *in vivo* including antiviral activities, cell growth regulation and tumorigenesis (Donze et al. EMBO J. 1995, vol. 14: 3828-34; Lee et al. Virology 1994, vol. 199: 491-6; Jagus et al. Int. J. Biochem. Cell. Biol. 1989, vol. 9: 1576-86).

The alpha subunit of protein synthesis initiation factor is responsible for binding the initiating methionyl-tRNA (Met-tRNA_{f}), together with a molecule of GTP, and placing Met-tRNA_{f} on native 40S ribosomal subunits (Pain, Eur. J. Biochem 1996, vol. 236; 747-771). During the course of this process GTP is hydrolyzed to GDP and inorganic phosphate, and when eIF-2 leaves the ribosome later in initiation (at the 60S subunit joining stage), it does so as an inactive eIF-2.GDP complex. Regeneration of active eIF-2 requires the exchange the GDP for a new molecule of GTP, catalyzed by the guanine nucleotide exchange factor eIF-2α (Pain, 1996). When eIF-2 becomes phosphotylated by PKR the initiation factor acquires an increased affinity for eIF-2α resulting in sequestration of the latter in an inactive complex. Consequently, the rate of guanine nucleotide exchange on both phosphorylated and unphosphorylated eIF-2 is decreased, as the concentration of available eIF-2α present in cell is reduced with respect to eIF-2, resulting in inhibition of polypeptide chain initiation. Not only does PKR activity have an effect on global rate of protein synthesis, but it may also selectively inhibit the translation of specific mRNAs that for various reasons have a greater than average requirement for active eIF-2 in the cell.

Regulation of protein synthesis through activated PKR arises from the interaction of PKR with double stranded RNA (dsRNA)- The activation of PKR by dsRNA depends on the concentration and size of the dsRNA- In particular, PKR is activated by low levels of dsRNA and inhibited by higher levels of dsRNA. This gives rise to a characteristic bell-shaped curve for activation of the enzyme as a function of dsRNA concentration (Clemens et al., Proc. Natl. Acad. Sci. USA 1975; 72(4): 1286-90; Levin DH. et al., Proc. Natl. Acad. Sci. USA 1980; 77(2):832-6 5). With respect to size of dsRNA, it has been found that molecules shorter than 30 base pairs (bp) in size fail to form a stable complex with PKR and do not activate the enzyme. Molecules longer than 30 bp bind and activate the enzyme, with an efficiency that increases with increasing chain length, reaching a maximum at about 85 bp-Analysis of complexes between dsRNA and PKR suggests that at maximal packing, the enzyme interacts with as little as a single helical turn of dsRNA (11 bp) but under conditions that allow activation, the binding site protects about 80 bp of duplex (Manche et al. Mol. Cell. Biol. 1992, vol. 12:5238-48).

WO-A-97/17456 discloses constructs capable of infecting mammalian cells comprising at least one semi-purified or pure SV40 capsid protein and a constituent selected from the group consisting of an exogenous DNA, a vector comprising an exogenous DNA, an exogenous RNA, a vector comprising an exogenous RNA, an exogenous protein or peptide product, and antisense RNA, ribozyme RNA or any RNA or DNA which inhibits or prevents the expression of undesired protein(s) in said mammalian cell and optionally further comprising operatively linked regulatory elements sufficient for the expression and/or replication of said exogenous protein in a mammalian cell. The protein product is preferably a therapeutic protein or peptide product which is not made or contained in mammalian cells, or is made or contained in such cells in abnormally low amount, or is made or contained in such cells in defective form, or is made or contained in mammalian cells in physiologically abnormal or normal amount and can be an enzyme, a receptor, a structural protein, a regulatory protein or a hormone. The invention further relates to a method for the in vitro construction of SV40 viruses or pseudoviruses constructs according to the invention. In a further aspect, the invention relates to mammalian, preferably human cells infected with the constructs of the invention or with constructs obtained by any of the methods of the invention. Still further, the invention relates to a method of providing a therapeutic DNA, RNA, protein or peptide product or antisense RNA to a patient in need of such product by administering to the patient a therapeutically effective amount of the SV40 viruses or pseudoviruses of the invention or a therapeutically effective amount of infected cells according to the invention. Pharmaceutical compositions comprising as active ingredient a therapeutically effective amount of the SV40 viruses or pseudoviruses according to the invention or a therapeutically effective amount of infected cells according to the invention are also within scope of this application.

WO-A-96/18733 relates to RNA molecules, including ribozymes, external guide sequences for RNAse P, and antisense oligonucleotides have been constructed which promote ribozyme cleavage of, or block transcription of, respectively, specific cancer-associated RNA, for example, acute promyeloleukocytic leukemia-associated RNA, follicular lymphoma-associated RNA and chronic myelocytic leukemia-associated RNA. Methods of producing and using such RNA molecules are also described.

The method of in vitro killing or inhibiting growth of a target cell of the present invention is defined in claims 1 and 2, the method of in vitro killing is defined in claims 3 and 4, the use of nucleic acid as a pharmaceutical is defined in claim 12, the use of a nucleic acid in the man facture of a medicament for treating a disorder is defined in claims 12, 13 and 14 the pharmaceutical composition is defined in claims 21, 22 and 23 the composition comprising an antisense RNA is defined in claim 28 the nucleic acid is defined in claim 29, the vector is defined in claim 30, the cell is defined in claim 33

Novel methods and compositions for selective killing of cells by activation of PKR are provided. In a preferred embodiment of the invention, a method is provided for causing cell death in a targeted population of cells that includes the steps of: selecting a nucleotide sequence at a single genetic locus in the targeted population that is absent from the equivalent locus in a population of non-targeted cells; obtaining one or more anti-sense RNA having sequence homology with the locus in the targeted population; permitting the anti-sense RNA to hybridize with an RNA transcribed from the locus in the targeted population so as to form a contiguous double stranded RNA for interacting with PKR. The activation of PKR gives rise to selective cell death in the targeted population.

In a further embodiment of the invention, a method is provided for treating a subject having a disorder characterized by proliferating cells, that includes selecting a nucleotide sequence at a single genetic locus in the proliferating cells that differs from the equivalent locus in a population of non-proliferating cells: administering an effective amount of a nucleic acid so as to provide anti-sense RNA having sequence homology with the locus in the proliferating cells, such that the resultant double stranded RNA has a length suited for activating PKR; and treating the disorder in the subject by selectively killing the proliferating cells.

In a further embodiment of the invention, a pharmaceutical composition is provided for treating proliferative disorders, comprising: an effective tumor cell growth inhibiting amount of anti-sense RNA having a nucleotide sequence with a length of at least 30 nucleotides that is complementary to contiguous mRNA sequence in a neoplastic cell, the mRNA being absent in a non-neoplastic cell, the resulting double stranded RNA in the neoplastic cell activating a double stranded RNA dependent protein kinase.

In a further embodiment of the invention, an anti-sense RNA is provided that includes a natural or synthetic molecule having a length of at least 35 nucleotides, and having a sequence that is capable of hybridizing *in situ* to a contiguous nucleotide sequence that spans a site specific mutation on a mammalian chromosome.

In a further embodiment of the invention, a nucleic acid is provided that includes a sequence that is a template for an anti-sense RNA, wherein the anti-sense RNA hybridizing to a messenger RNA, the messenger RNA being transcribed from a contiguous nucleotide sequence that spans a site specific mutation on a mammalian chromosome, the mutation occurring in a neoplastic cell and absent in a non-neoplastic cell, the nucleic acid having a length of at least 30 nucleotides, and more particularly 85 nucleotides, and no greater than 100 nucleotides.

In a further embodiment of the invention, a vector is provided more particular a retroviral vector, more particularly a lentivirus vector that includes a sequence of nucleotides that is a template for an anti-sense RNA of a length greater than about 30 nucleotides and less than 100 nucleotides, more particularly in the range of 35 through 85 nucleotides, wherein the anti-sense RNA hybridizes under stringent conditions to a messenger RNA, the messenger RNA being transcribed from a contiguous nucleotide sequence that spans a site specific mutation on a mammalian chromosome, the mutation being a distinguishing characteristic of a neoplastic cell, the mutation being absent on non-neoplastic cells.

In a further embodiment of the invention, a cell population is provided which is transfected with a vector according to the above description. More particularly, the transfection is a stable transfection and the cell population is maintained in cell culture medium.

### Brief Description of the Figures

Figure 1 shows the mechanism of specific activation of PKR in U87MGΔEGFR cells.
Figure 2 shows the expression of EGFR mRNA in glioblastoma cells analyzed by RNAse protection analysis. Expression of mutant and wild type forms of EGFR mRNA in U87MGΔEGFR (1) and U87MG (2) are demonstrated using RNase protection analysis. The bands on the gel correspond in descending order to (A) actin mRNA; (B) Δ2-7 EGFR mRNA and (C) wt EGFR mRNA.
Figure 3 shows the effect of IFN-α on expression of PKR in U87MGΔEGFR cells on Western blots. Cells were treated for 18 hours with varying concentrations of IFN-α and then lyzed. The gel shows the effect of adding 0,1,10, 50 and 100 U/ml of α-interferon.
Figure 4 demonstrates the effect of dsRNA on survival of glioblastoma cells. U87MG cells(white) and U87MGΔEGFR cells(black). Cells were transfected with polyI-C as indicated in the presence or absence of α-interferon and the controls were untreated cells
Figure 5 shows the expression of anti-sense RNA in glioblastoma cells (1) transfected U87MGΔEGFR (2) transfected U87MG. (3) untransfected U87MGΔEGFR and (4) untransfected U87MG where both (1) and (2) were transfected with pΔEGFR-AS plasmid Expression of anti-sense RNA was detected by RNase protection analysis.
Figure 6 shows the effect of transfecting glioblastoma cells three times with anti-sense RNA expressing plasmid as measured by percent survival of cells.
Figure 7 shows the effect on glioblastoma cells (U87MGΔEGFR cells and U87MG cells) of transfection with anti-sense RNA expressing plasmids as visualized by light microscopy.
Figure 8 shows the effect of triple transfection with one or a combination of plasmids as indicated on survival of glioblastoma cells. (A)(i) U87MGΔEGFR and (A)(ii) U87MG. (B) U87MGΔEGFR cells after the third transfection.

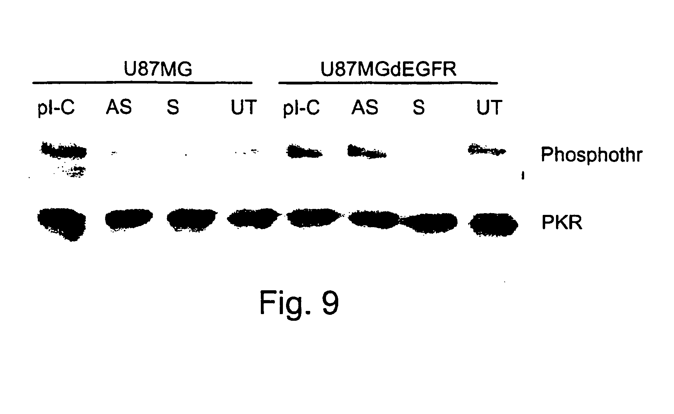

Figure 9 shows activation of PKR in glioblastoma cells treated with 500 U/ml of IFN-α by Western blot analysis of lyzed cells. (UT) untreated cells; p-IC cells transfected with PolyI-PolyC; (AS) pΔEGFR-AS; (S) pΔEGFR-S. (AS=antisense, S=sense)
Figure 10 shows the effect of pΔEGFR-ASRNA on growth of glioma cells (a) effect of infection of pΔEGFR-ASRNA expressing vector on growth of glioma cells in a monolayer. Percentage surviving cells was calculated relative to untreated cells (UT); (b) effect of pΔEGFR-ASRNA on growth of glioma cells in soft agar.
Figure 11 shows (a) activation of PKR in glioblastoma cells. Cells either untreated (UT), transfected with pI-C, or infected with a vector and treated with 2-AP (5mM final concentration) as indicated. (b) infection of AS RNA expressing vector on total translation in glioblastoma cells. The graph shows fold inhibition of total protein/total mRNA ratio with respect to untreated (UT) cells.
Figure 12 shows the rescue of the U87MGΔEGFR cells by PKR inhibitors. (a) Effect of 2-Aminopurine on survival of cells.
Figure 13 shows the effect of IFN-α on PKR expression for three lymphoma cell lines-Lam, Nalm-6 and Karpas 299. A western blot shows enhanced PKR expression in the presence of interferon.
Fig. 14 shows *in vitro* activation of PKR in karpas 299 lymphoma cells showing the levels of phosphorylated eIF2α/PKR was greatest at 10µg/ml of Poly I-C.
Figure 15 (a) shows activation of PKR in lysates of Karpas299 treated with IFN-α and (b) in the absence of IFN-α.

### Detailed Description of Specific Embodiments

Embodiments of the invention are directed to selectively killing target cells. For clarification, terms have been defined for use in the description and the claims except where the context requires otherwise. Accordingly, "target cells" are defined as any cell involved in a pathologic condition that has an identified mutation even where the expression product of the mutated site is unknown. Examples of target cells are cancer cells more particularly neoplastic cells and metastasizing cells. Additional examples of target cells that are associated with disease in animals (including humans) include: skin cells in psoriasis and keloid scarring; vascular disorders including atherosclerosis and restenosis; vasculogenesis and angiogenesis, for example diabetic retinopathy. Target cells may include infectious agents including eukaryotic organisms, for example; protozoa and mycoplasma. Additionally, target cells may include selected cells of the immune system, for example, autoimmune T-lymphocytes.

The neoplastic cells of many cancers have acquired chromosomal translocations and deletions. (See Table 1) Such chromosomal rearrangements and truncations lead to the production of unique mRNA species which are the result of alternative splicing of pre-mRNA. Some genes express as a single truncated form of mRNA or mRNAs formed when two genes are fused after translocation. The term "single transcript" or "unique transcript" may be defined as a contiguous sequence associated with a mutation in contrast to two or more smaller transcripts that arise in the absence of a mutation.

The term "antisense RNA" includes any oligonucleotide that is capable of hybridizing to mRNA in the target cell and triggering double stranded RNA dependent protein kinase. The oligonucleotide may be a chimera between RNA and DNA. The oligonucleotide may be modified in the base moiety, the sugar moiety or the phosphate backbone for example, to improve its stability. The oligonucleotide may include appended groups such as peptides or agents facilitating transfer across membranes. Examples of modified nucleotides include thos known in the art , examples of which are provided in Patent 5,955,306 herein incorporated by reference.

Selective killing of cells is achieved by activating double stranded RNA dependent protein kinase (PKR) through the formation of double stranded RNA of sufficient length to activate the enzyme only in cells with a mutation. In a preferred embodiment, this is achieved by introducing at least one anti-sense oligonucleotide into a population of cells either *in vitro* or *in vivo* by any method known in the art. This includes the use of vectors such as plasmids, DNA or RNA virus vectors, liposome encapsulated nucleic acids or naked DNA. Examples of DNA virus vectors include but are not limited to vaccinia virus, herpes virus and adenovirus vectors. Examples of RNA virus vectors include retroviruses including lentiviruses. The virus vectors may be engineered to achieve an infection efficiency of up to 100% (see Examples 1 and 6). Alternatively, the nucleic acid may be administered within a hydrophilic excipient material (US Patent 5,994,314). The anti-sense RNA encoding sequences may be introduced into the host genome and stable expression of anti-sense RNA obtained. Expression of anti-sense RNA may be enhanced placing the anti-sense sequence under inducible or consitutive strong promoters, for example the U6 small nuclear RNA promoter. Early transcription termination signals in the anti-sense RNA are avoided or removed. The nucleic acid may be but is not required to be specifically targeted to selected cells because the mode of action is such that only targeted cells will form double stranded RNA of sufficient length to activate PKR.

Replicated anti-sense RNA reagent, produced after transfection or infection of target and non-target cells and target cells or target cells only, hybridizes substantially completely with mRNA transcribed from a pre-selected genetic locus in the target cells. The replicated anti-sense RNA may be a single full length molecule or alternatively may be more than one smaller anti-sense molecules which nonetheless collectively hybridize to messenger RNA in the target cell to form a single double stranded (ds) molecule. The messenger RNA is transcribed from a genetic locus that differs in sequence from the equivalent locus in non-target cells by a mutation in the sequence. A mutation is defined here as any of a substitution, a translocation, a deletion or insertion, and a rearrangement where one or more bases are involved. The intact double stranded RNA formed by hybridization of anti-sense RNA and messenger RNA in the target cells is of a length suited for activating PKR and bringing about cell death. The length of double stranded RNA suitable for activating PKR is at least 30 nucleotides, and generally less than 120 nucleotides in size, more particularly less than 100 nucleotides with an improved effect when the length is between 35 and 80 nucleotides. In non-target cells, the length of dsRNA produced upon hybridization is insufficient to activate PKR because the complementary region is not contiguous in the presence of non mutated sequence. Consequently, cell death is not triggered by PKR in those cells.

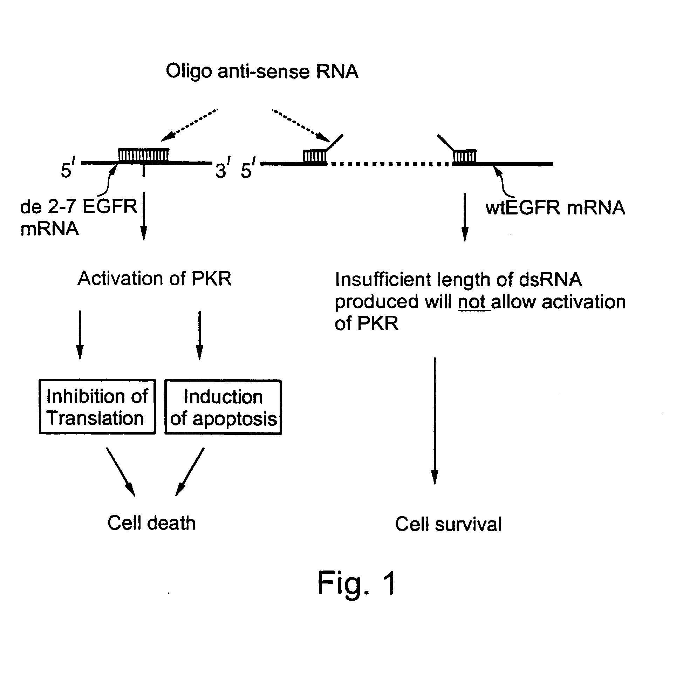

Figure 1 is a diagrammatic representation of how anti-sense RNA fragments may hybridize to messenger RNA to form a contiguous double stranded sequence of 30-85 nucleotides in the target cells so as to give rise to the inhibition of translation and to aptoptosis. In contrast, in wild type cells, the anti-sense RNA fragments hybridize to messenger RNA at distant sites and therefore produce two distinct and substantially shorter double stranded RNA molecules. These short fragments do not effectively activate PKR in non-target cells and therefore cell death is not triggered in these cells. To identify cancer cells having a mutation which may be used to activate PKR and thereby give rise to apoptosis, the expression of PKR and PKR activity status may be examined prior to the implementation of the dsRNA killing strategy ("DKS"). In order to implement DKS, any unique mRNA molecule may be targeted with a vector containing complementary nucleotide sequences or with naked oligonucleotides, including those sequences which lack a defined function. The dsRNA is generated *in situ* following a binding event between the oligonucleotides introduced into the cell or made in the cells from template introduced into the cell:

The pharmaceutical composition for administering the oligonucleotides may be formulated using one or more physiologically acceptable carriers or excipients depending on the route of administration. It is envisaged that the pharmaceutical composition may be administered by any technique known in the art including oral administration, administration by injection, transdermal administration or administration through the mucosal surface. Examples of formulations that may be used are provided in USP 5,955,306 incorporated by reference.

Thus the approach described here is applicable to the treatment of a wide range of cancers and other proliferative disorders in which chromosomal translocation and truncation mutations occur (Table 1). Support for the general approach described in Figure 1 is provided in the examples which are discussed below. The examples are not intended to be limiting.

In an embodiment of the invention, an interferon was added to further enhance the activation of dsRNA dependent protein kinase in the presence of double stranded RNA (Example 5). Whereas any interferon can be used, the enhancement effect is exemplified using alpha interferon. This approach may be used in the treatment of cell proliferative disorders in subjects including neoplastic disorders exemplified in Table 1.

In certain embodiments of the invention, the homology of antisense RNA for mRNA may be determined *in vitro* under stringent conditions of hybridization. The stringent conditions for hybridization include those that are generally accepted in the art such as described in chapter 6 of "A Compendium of Methods from Current Protocols in Molecular Biology" 1992 ed. Asubel et al. pub. John Wiley & Sons, Inc. Alternatively, effective hybridization is determined *in situ* between antisense RNA and mRNA as determined by a biological assay in which PKR activity is measured.

In a preferred embodiment of the invention, a pharmaceutical composition for treating proliferative disorders is provided that utilizes a vector or naked DNA to generate anti-sense RNA in the target cell for binding messenger RNA so as to cause double stranded DNA of sufficient length to be formed and to activate PKR and bring about cell death. The composition may be administered in a pharmaceutically acceptable non-toxic formulation suited for providing access to the target cells and minimizing breakdown of the composition either in storage or incurred during administration. This formulation may include encapulation within liposomes or absorption to microbeads. The composition may also be modified to provide an effective clearance profile in the subject. The composition may be administered by any one of a number of routes including transdermally, orally, or by intravenous, intramuscular or transmucosal means. It may be in liquid form and as a spray. An effective amount of an anti-sense RNA is defined to be sufficient to cause cell death to a significant number of target cells so as to be beneficial to the patient with respect to disease outcome or symptom alleviation. The pharmaceutical composition may include additional agents to enhance the therapeutic effect of the formulation so as to enhance activation of double stranded RNA dependent protein kinase and subsequent cell death.

Embodiments of the invention are exemplified by lymphomas. A number of recurring cytogenenetic abnormalities have been identified in various lymphomas. These abnormalities have been found to correlate with clinical, morphological and immunophenotypic characteristics (reviewed in Ong and Beau, 1998 Seminars in Oncology 25: 447-460). For example, in 70%-80% of human follicular lymphoma (FL) cases, a non-Hodgkin's lymphoma subtype is characterized by a t(14;18) chromosomal translocation that joins the 3' non-coding region of the antiapoptotic gene *bcl-2* and the immunoglobulin heavy chain locus (*bcl-2*/*IgH*) (Tsujimoto et al., 1984; Bakhshi et al., 1985). As a result, these lymphomas express elevated levels of both *bcl-2*/*IgH* chimeric transcript and Bcl-2 protein (Cleary et al., 1986, Cell 47: 19-28) which possess survival advantages associated with neoplastic transformation (Cory, 1986: Advance in Cancer Research 47: 19-28). Another lymphoma subtype is large anaplastic transformation (ALCL). More than half of these lymphomas possess the chromosomal translocation t(2:5), that leads to the expression of a fusion protein comprised of the amino-terminal portion of the nuclear phosphoprotein nucleophosmin (NPM) and the cytoplasmatic domain of anaplastic lymphoma kinase (ALK) (Morris et al., 1994 Science 263: 1281-1281).

In another example, glioblastomas have mutations arising from deletions and translocations. Examples 1-6 describe how antisense RNA can give rise to double stranded RNA which activates PKR bringing about apoptosis in target cells.

**Table 1. Genetic Abnormalities in Cancers**

| Phenotype | Rearrangement | Involved Genes | Reference |
|---|---|---|---|
| B-cell NHL | | | |
| BL | t(8;14)(q24;q32) | MYC, IGH | Ong et al. Semin. Oncol 1998;vol 25:447-60 |
| | t(2;8)(p12;q24) | IGK, MYC | |
| | t(8;22)(q24;q11) | MYC, IGL | |
| FL, DCLL | t(14;18)(q32;q21) | IGH, BCL2 | Ong et al. |
| DLCL | t(3:22)(q27;q11) | BCL6, IGL | Ong et al. |
| | t(3;22)(q27;q32) | BCL6,IGL | |
| | t(3q27) | BCL6 | |
| MCL | t(11;14)(q13;q32) | CCND I,IGH | Ong et al. |
| LPL | t(9;14)(p13;q32) | PAX5, IGH | Ong et al. |
| SLL | t(14;19)(q32'q13.3) | IGH,BCL3 | Ong et al. |
| MALT | t(11;18)(q21;q21) | | Ong et al. |
| Variable | t(1;14)(p22;q32) | | Ong et al. |
| | t(1;14)(q22;q32) | | |
| | t(3;14)(p21;q32) | | |
| | t(10;14)(q24;q32) | LYT10,IGH | |
| | t(11;14)(q23;q32) | RCK,IGH | |
| | t(11;14)(q23;q32) | LPC,IGH | |

| T-cell NHL | | | |
|---|---|---|---|
| ALCL (CD30+) | t(2;5)(p23;q35) | ALK, NPM | Ong et al. |
| CTCL | r(10q24) | LYT10 | Ong et al. |
| Variable | t(7;14)(q35;q11) | TCRB, TCRA | Ong et al. |
| | t(11;14)(p13;q11) | RBTN2, TCRD | |
| | inv(14)(q11q32) | TCRA, TCL I | |
| | t(14;14)(q11;q32) | | |

| Leukemia | | | |
|---|---|---|---|
| CML | t(9;22) | BCR, ABL | Shtivelman et al. Nature 1985; 315:550.4 |
| AML | t(7;11)(p15;p15) | NYP98, HOXA9 | Kwong et al. Genes-Chromosome-Cancer 1999; 25:70-4 |

| Other Cancers | | | |
|---|---|---|---|
| Multiple liposarcoma | t(12;16)(q13;p11) | TLS/FUS, CHOP | Schneier-Stock, etal. Cancer-Gene-Vytogenet 1999; 111:130-3 |
| Clear cell sarcoma | t(12;22)(q13;q12) | EWS, ATFI | Pellin, et al. Genes-Chromosomes-Cancer, 1998; 23:358-60 |
| DSRCT | t(11;22) | EWS, WT1 | Shimizu, et al. Cancer-Genet-Cytogenet. 1998; 106: 156-8 |
| Synovial sarcoma | t(X;18)(p11;q11) | SYT-SSX1 | Kawai et al. N.Engl.J.Med. 1998; 338:153-60 |
| Thyroid cancer | PTC5 | RET, RFG5 | Klugbauer et al. Cancer-Res. 1998; 58(2): 198-203 |
| Neuroblastoma, Ewing cancers | t(11.22)(q24.q12) | EWS, FLI1 | Burchill, et al. Eur. J. Cancer. 1997; 33(2):239-43 |
| Myoxoid chondrosarcoma | t(9;22)(q22;q12) | EWS, TEC | Brody, et al. Am. J. Pathol. 1997; 150(3): 1049-58 |
| Alveolar rhabodomyosarco ma | 13q with either 2q35 or 1p36 | FKHR, PAX7/PAX3 | Weber-Hall, et al. Genes-Chromosomes-Cancer. 1996; 17(1): 7-13 |
| Prostate carcinoma | t(6;16)(p21;q22) | TPC/HPR | Veronese et al. Cancer-Res. 1996; 56(4): 728-32 |
| Gastric carcinoma | 11q23 | Duplication of ALL-1 | Baffa et al. Proc. Natl. Acad. Sci, USA. 1995; 92(11): 4922-6 |
| Lung cancer | 3p21.3 | Alternative splicing of RBM6 | Timmer et al. Eur. J. Hum. Genet. 1999; 7(4): 478-86 |
| Breast and ovarian cancers | | Alternative splicing of TSG101 | Carney et al. J. Soc. Gynecol. Investig. 1998; 5(5): 281-5 |
| Pancreatic carcinoma | 3p14 | Alternative splicing of FHIT | Simon et al. Cancer Res 1998; 58(8): 1583-7 |
| Colorectal cancer | | Alternative splicing of hMSH2 | Xia et al. Cancer Res 1996; 56(10): 2289-92 |
| Wilms', kidney and thyroid tumors | | Alternative splicing of PAX8 | Poleev et al. Eur J Biochem 1995; 228(3):899-911 |
| Retinoblastoma | | Deletion of RB | Hashimoto et al. Onocogene 1991; 6(3): 463-9 |
| Glioblastoma | | Deletion of EGFR | Sugawa et al. Proc. Natl. Acd. Sci. U.S.A. 1990; 87(21): 8602-6 |

| | | | |
|---|---|---|---|
| 1. References indicate fusion of specified genes. 2. Abbreviations: NHL non-Hodgkin's lymphoma; BL Burkitt's lymphoma; FL follicular lymphoma; DLCL diffuse large cell lymphoma; MCL mande cell lymphoma, LPL lymphoplasmacytoid lymphoma, SLL small lymphocytic lymphoma; MALT mucosa associated lymphoid tissue; ALCL anaplastic large cell lymphoma; CTCL cutaneous T cell lymphoma; DSRCT desmoplastic small round cell tumor; CML chronic myeloid leukemia; AML acute myeloid leukemia. | | | |

The references in Table 1 describe published data on mutations for certain malignancies. The locations of the mutations and their sequences are readily accessible to the person of ordinary skill in the art through publicly available databases. Those sites provided in Table 1 are given as examples. The invention is not intended to be limited by these examples. Exemplary sequences associated with selected deletions described in Table 1 are provided below (AS RNA denotes anti-sense RNA and "Translocation" refers to the mutation on the DNA).

### FL,DCLL T(14,18) (Q32;Q21) IGH,BCL2

SEQ ID No 1: 3'-GUUUUCCUAAGACUCUUCCACUCUAUUCUUGACUCA-5' AS-RNA (36 bases)
SEQ ID No 2:
   5'-CAAAAGCATTCTGAGAAGGTGAGATAAGAACTGAGT-3' Translocation

### LPL T(9;14) (P13;Q32) PAX5, IGH

SEQ ID No 3:
   3'-ACUUAAAAUAAAAAAAACUUCCCCUGAAUCACUACAGACA AS-RNA (40bases)
SEQ ID No 4:
   5'-TGAATTTTATTTTTTTTGAAGGGGTCTTAGTGATGTCTGA Translocation

### ALCL (CD30+) T(2;5) (P23;Q35) ALK,NPM

SEQ ID No 5:
   3'-CUGUUAACUACUGGACCUUCACAUGGCGGCCUUCGUGGUC AS-RNA (40bases)
SEQ ID No 6:
   5'-GACAATTGATGACCTGGAAGTGTACCGCCGGAAGCACCAG Translocation

### Glioblastoma Deletion in EGFR

SEQ ID No 7:
   3'-CCGAGACCUCCUUUUCUUUCCAUUAAUACACCACUGUCUA AS-RNA (40bases)
SEQ ID No 8
   5'-GGCTCTGGAGGAAAAGAAAGGTAATTATGTGGTGACAGAT Translocation

The detection of these and other recurring abnormalities are useful for diagnosis and prognosis assessment leading to treatment with anti-sense RNA to trigger cell death.

### Examples

### Example 1: Targeted Cell killing of Glioblastoma cells

### Formation of plasmids for transfection into target cells.

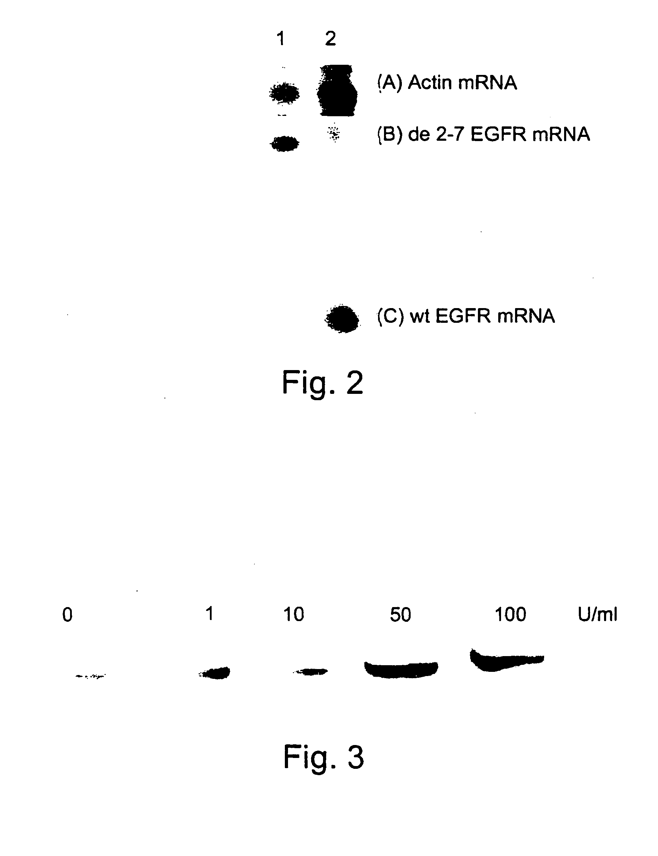

The U87MGΔEGFR cell line (Nishikawa R et al.; (1994) Proc. Natl. Acad. Sci. USA 91: 7727-31) which was created by infection of human glioblastoma cells U87MG with amphotropic virus carrying EGFR gene deleted for exons 2-7, expresses truncated form of EGFR (Δ2-7EGFR) and the U87MG cell line (Ponten J., et al., (1968) Pathol. Microbio. 'I Scand.; 74:465-86) expresses wild type (wt) form of EGFR. This was determined by RNase Protection Analysis that showed expression of wild type EGFR mRNA in both U87MG (parental) and U87MGΔEGFR cells, while mutant mRNA was expressed only in U87MGΔEGFR cell line (Figure 1). The level of mutant mRNA was several times higher than wt mRNA. The amount of wt mRNA (U87MG) to mutant mRNA (U87MGΔEGFR) was determined with respect to levels of actin in each sample. (Figure 2). These cell lines provided a suitable model system to determine whether PKR could be selectively activated in target cells as schematically represented in Figure 1.

### Anti-sense RNA expressing plasmid

To generate high levels of anti-sense RNA in the cell, the EGFR anti-sense sequence was cloned into a U6 expressing construct (He et al. (1998) J. Natl. Cancer. Inst.:1080). In this construct, a U6 small nuclear RNA promoter controls expression of the subcloned gene by RNA polymerase III.

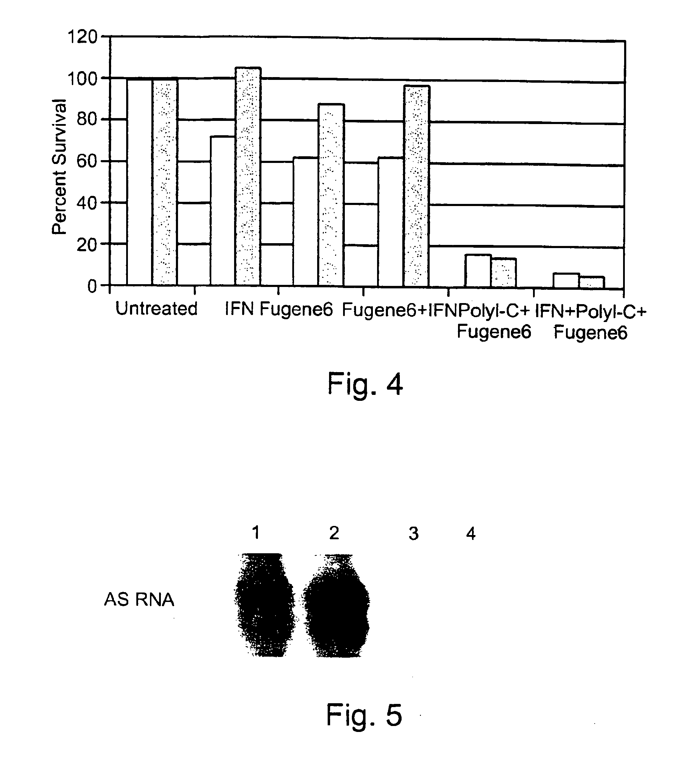

The Δ2-7 EGFR mRNA has a deletion of 801 nucleotide (nt) of the coding sequence of the extracellular domain (Kawai, A. et al. (1998) J. Med. 338: 153-60). This mutant is the most virulent form of glioblastoma. It is capable of expressing anti-sense RNA that is complimentary to short fragments of EGFR mRNA flanking the deletion region. (Figure 5 shows the expression of AS RNA in glioblastoma cells). The AS RNA completely hybridizes with Δ 2-7 EGFR mRNA yielding a double stranded molecule that is of sufficient length to activate PKR. In contrast, wild type EGFR mRNA undergoes hybridization with only half of the AS RNA yielding dsRNA with insufficient length to activate PKR. Once activated PKR was found to selectively inhibit growth of U87MGΔEGFR cells.

### The oligonucleotides

Vectors were constructed for expression of 39-bp long anti-sense and sense oligonucleotides corresponding to fragments of Δ2-7 EGFR mRNA flanking the deletion region. These vectors were synthesized and cloned into *Xh*o I and *Nsi* I sites of the U6 expression plasmid. In this construct, the U6 small nuclear RNA promoter controls transcription of the subcloned gene by RNA polymerase III, generating high levels of short RNA. The anti-sense transcript is complementary to short fragments of mutant EGFR mRNA flanking the deletion region. Plasmid p? EGFR was designed so that complete hybridization of the AS RNA with ?(2-7) EGFR mRNA would yield a double stranded molecule with sufficient length to activate PKR. The expression of anti-sense RNA was tested by RNase Protection Analysis following transfection of the cells (Figure 4). Sequences of the inserts are as listed below and were verified by sequence analysis.
SEQ ID No 9
pΔEGFR-AS: TCTGTCACCACATAATTACCTTTC**g**TTTCCTCCAGAGCC
SEQ ID No 10
pΔEGFR-S : GCTCTGGAGGAAAAGAAAGGTAATTATGTGGTGACAGAT

The transcription termination signal for RNA polymerase III is a string of 4 or more thymidine residues (He et al. (1997) Cancer Res. 57:3993-9). To prevent early termination of transcription, thymidine in position 25 of pΔEGFR-AS was replaced for guanine (bold). Since it was shown that binding and activation of PKR by dsRNA can tolerate mismatch of single pair of nucleotides (Kunkel et al. (1989) Nucleic Acid Research, 17:7371-9), this replacement did not interfere with PKR activation. A 39 nt oligonucleotide was utilized in preference to a 40nt to avoid producing a transcript having an extra sequence of *Xho* I site that could hybridize with wt EGFR mRNA generating a dsRNA with 24 bp length. A dsRNA of 24 bp is potentially capable of activating PKR which would compromise the selectivity of the PKR for target cells.

### Example 2: Transfection of cell lines with plasmids

The cells of human glioblastoma line U87MG (Ponten et al. (1968) Acta Pathol. Microbio.' I Scand, 74: 465-86) which express wild type EGFR only were maintained in Dulbecco's modification of Eagle medium (DMEM) (Beith-Ha-Emek, Israel) supplemented with 10% fetal calf serum and antibiotics. The cells of U87MGΔEGFR line (Nishikawa et al. (1994) Proc. Natl. Acad. Sci. USA, 87:8602-6), which express both wild type and truncated form of EGFR were maintained in the same conditions plus 400µg of G418 per ml were added to the medium.

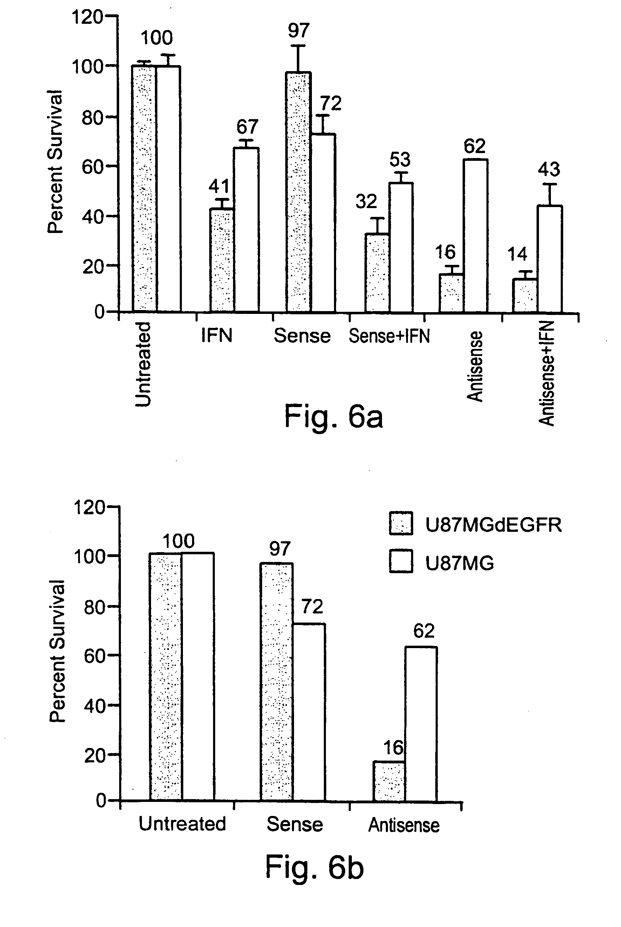

Transfections either of DNA or dsRNA (polyI-polyC (polyI-C), Pharmacia) were performed using FuGene 6 Transfection Reagent (Boehringer Mannheim) according to manufacturer's instructions. Average transfection efficiency was determined as 30% by transfection of β-galactosidase encoding plasmid and subsequent β-galactosidase staining of the cells. Alternatively, U87MG and U87MGΔEGFR cells seeded in 96 well dishes at a density of 2000 cells/well were transfected three times at 48 hour intervals with pΔEGFR-AS/S plasmids (0.1µg) and treated continuously with IFN-α (100U/ml) to determine effect on cell growth (Figure 6). Transfection with pΔEGFR-AS into U87MGΔEGFR cells resulted in only 16% survival compared to the untreated cells, while effect on survival of U87MG cells was much less dramatic and is comparable to effect of transfection with pΔEGFR-S (62 and 72% respectively) (see Figure 6).

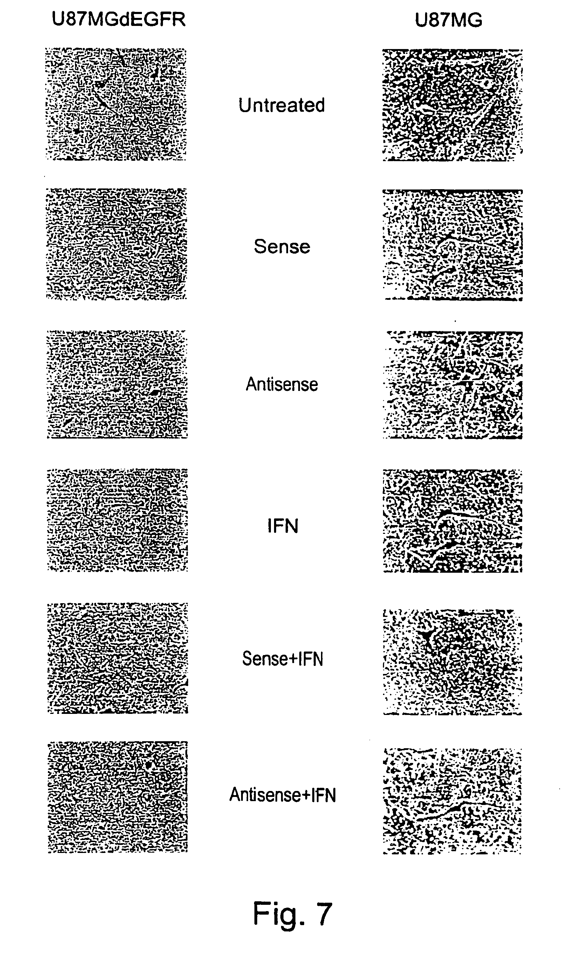

Transfection efficiency was evaluated by co-transfecting cells at a density of 10,000 cells/well in a 24 well plate with pΔEGFR-AS/S plasmids (1.5µg of each) containing β-galactosidase coding sequences, growing overnight and staining the cells with 0.5 µg β-galactosidase. The results of this experiment captured by digital camera on a microscope were in good agreement with the results of triple transfections pΔEGFR-AS to glioblastoma cells showing the strong effect of anti-sense RNA on U87MGΔEGFR cells and virtually no effect on U87MG cells (Figure 7).

To increase transfection efficiencies of the plasmids (single transfection efficiency-30%), antisense/sense RNA expressing lentiviral vectors with high infection efficiency (100%) were constructed. These viral vectors may also be in gene therapy of human subjects. For lentiviral vector production, the U6 cassette was excised from pΔEGFR-AS/S by BamHI/EcoRI digestion, filled in and ligated with SIN-PGK transfer vector (Zufferey, R. et al. (1987) Nat. Biotechnol., 15:871-5) which was digested with XhoI and filled in. Clones with insert orientation that preserves the same direction of the transcription from 5'LTR of the plasmid and the U6 promoter were selected. Virus was obtained by transient co-transfection of the constructed plasmids with pMD.G and pCMVDR8.91 plasmids into the 293T cell line (Zufferey et al.(1997); Naldini, L., (1996) Science 272:263-7). The expression of antisense and sense RNA was verified by RNase protection analysis following infection of the cells.

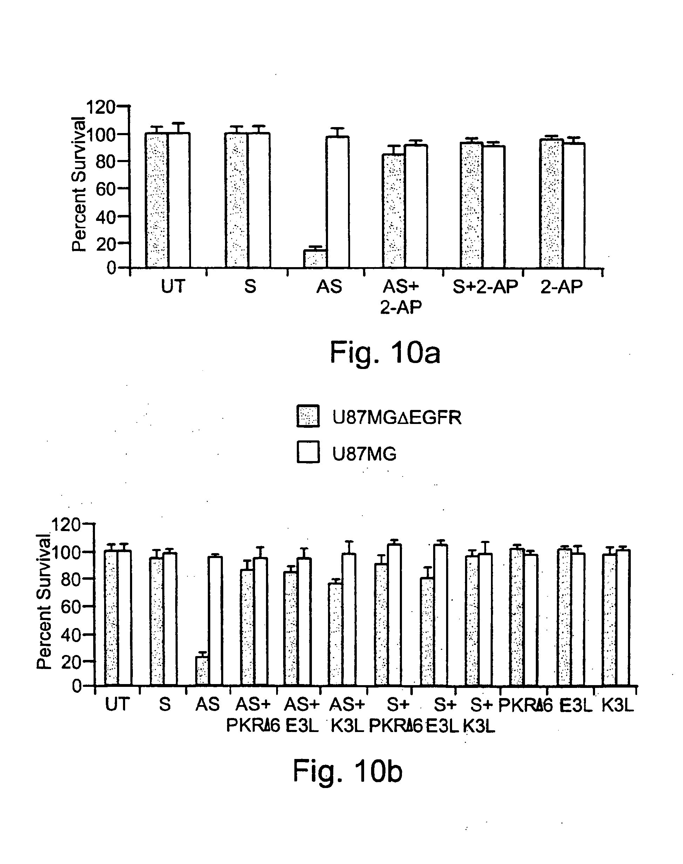

Single infection of the antisense expressing vector resulted in the death of more than 90% of U87MGΔEGFR cells grown in monolayer while no significant effect was observed on either U87MG cells or U87MG cells over-expressing the wild type EGFR (Fig. 10a). A similar effect was observed on cells grown in soft agar (Fig. 10b). Growth inhibition did not result from the reduction in expression of truncated EGFR since this receptor does not enhance proliferation of the cells grown *in vitro.*

### Example 3: Detection of mRNA levels in cells

Messenger RNA levels were measured by RNase protection analysis. Human glioblastoma cells were seeded at a density of 1x10⁶ per 6-cm diameter petri dish and grown overnight and transfected with two µg of either pΔEGFR-AS or pΔEGFR-S containing plasmid using Fugene 6 Transfection Reagent (Boehringer Mannheim). Total RNA was isolated from either transfected and untransfected cells at 24 hours after transfection using RNA Pure System (Boehringer Mannheim) according to the manufacturer's instructions. 10µg of RNA were hybridized with appropriated probe and RNase protection analysis was performed using RPA II Kit (Ambion Inc., USA) according to manufacturer's instructions.

Hybridization probes were prepared as follows: pΔEGFR-AS and pΔEGFR-S plasmids were digested with BamHI and riboprobes containing complementary sequences to either anti-sense or sense RNA were transcribed from SP6 promoter using Riboprobe System-SP6 (Promega). Riboprobe complementary to sense RNA was used to detect EGFR mRNAs levels in the cells. This probe protects one 39 nt fragment of mutant EGFR mRNA and two fragments (20 and 19 nt) of wild type EGFR mRNA, flanking the deletion region.

### Effect of dEGFR anti-sense RNA on protein synthesis

PKR was demonstrated to be activated upon expression and hybridization of ΔEGFR anti-sense RNA with target mRNA so as to inhibit protein synthesis. Global translation levels were measured in infected and uninfected U87MG and U87MGΔEGFR glioblastoma cells as well as the translation efficiency of mutant EGFR mRNA after infection of the cells with anti-sense RNA vector and treatment with α-interferon.

The translation levels are determined by the ratio of total protein level to total mRNA level and the ratio of EGFR protein level to EGFR mRNA level in both mutant and wild type.

Determination of protein concentration was carried out as follows: Cells were lysed using 40 µl of Lysis buffer 1. 10µl of lysates were then transferred to small pieces of Whatman blotting paper, as were BSA standards dissolved in the same buffer. The samples were stained with Coomassie blue for 30 min. using 30 ml of staining buffer (2.5 g/L of Coomassie brilliant blue G-250 (BDH), 40% methanol and 10% acetic acid). Samples were then extensively washed with washing buffer (1% glycerol, 20% methanol, 7% acetic acid) and transferred to 24 well plates. The color was extracted with 1ml of 3% SDS. Duplicates of 200 µl of the obtained solutions were transferred to 96 well plate and the optical density was read in an ELISA reader at 595 nm. Concentration of the protein was calculated using BSA concentrations as a standard.

### Example 4: Visualization of stained cells by light microscopy

Methylene blue staining of the cells: Cells were seeded into 96-wells plate and grown overnight. After appropriate treatment, cells were fixed with glutardehyde at 0.5% of final concentration. Cells were then washed three times with double distilled H₂0 and one time with 200µl of Borate Buffer (0.1 M, pH 8.5). Cells were than stained with 200ul of 1% Methylene Blue resolved in Borat Buffer. After intensive washing and drying, the color was extracted with 200µl of 0.1 M HCI for one hour in 37°C and the optical density was read in ELISA reader at 630 nm.

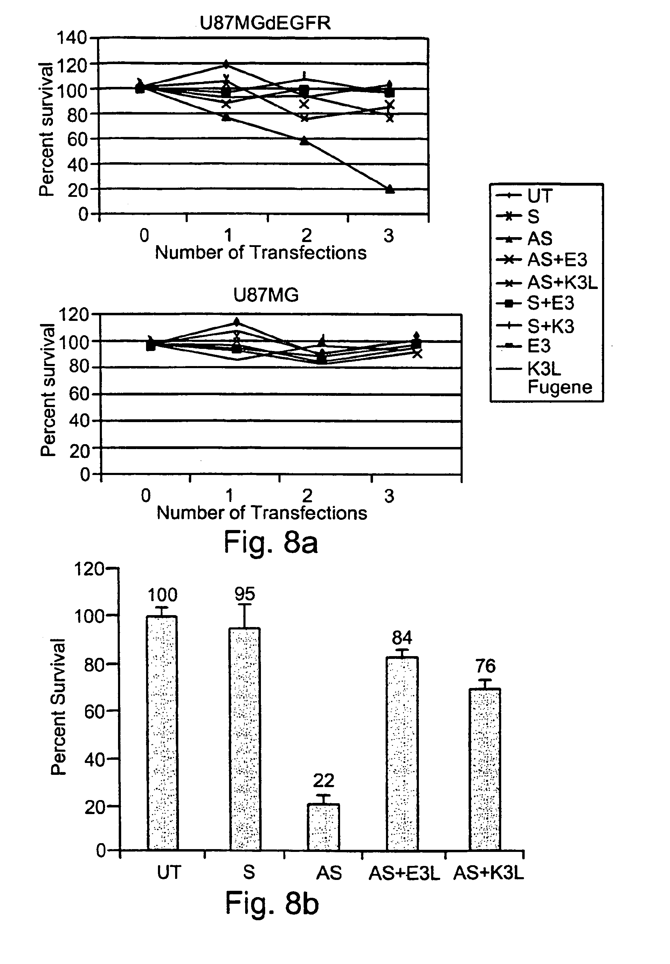

Figure 8 shows the results of methylene blue staining of the cells performed 48 hours after the last transfection. Percent survival of the cells was calculated regarding to untreated cells. Graphs A and B depict the results of the same experiment where B does not include the effect of IFN-α. (Figure 8)

Alternatively, cells were stained with β-galactosidase. Cells were seeded into 24 well at density of 10,000 cells per well and grown overnight. After appropriate treatment, cells were washed with PBS and fixed with Fixation Buffer (2% Formaldehyde, 0.2% Glutardehyde diluted in PBS) for 5 min. at 4°C. The cells were then washed twice with PBS and incubated overnight in 1 ml of Staining Buffer (1 mg/ml X-gal, 5mM K-ferricyanide, 5 mM K-ferrocyanide, 2mM MgCl₂in PBS, pH7.4). The colored cells were detected by light microscopy.

### Example 5: Measurement of PKR expression

Cells were seeded at a density of 5x10⁵ per 6-cm diameter petri dish and grown overnight. Cells were then infected with appropriate vector and treated with 2-aminopurine (Sigma) where indicated. PolyI-C (25 µg/ml) was transfected using FuGene6™ transfection reagent (Roche) 1 hour before lysis. Sixteen hours after infection cells were lysed with 200 µl of Lysis Buffer 1 (20 mM Tris-HCl pH 7.5, 5 mM MgCl₂, 50 mM KCI, 400 mM NaCl, 2mM DTT, 20% Glycerol, 20 mM β-glycerophosphate, 100 mM NaF, 10 µg/ml PMSF, 10 µg/ml Aprotinin, 1% Triton X-100) and the samples were centrifuged at 12,000xg for 5 min. at 4°C to remove cell debris. Fifty µg of each sample were taken for Western blot to determine phosphorylation of endogenous eIF-2α. For determination of PKR activity 80 µg of each sample were incubated for 20 min at 37°C with 0.5 µg of recombinant eIF-2α (Sood, et al. (2000) Genetics. 154:787-801) in reaction buffer (20 mM Tris-HCl pH 7.5, 2 mM MgCl₂, 2 mM MnCl₂ 50 mM KCI, 5% Glycerol, 10 mM NaF, 100mM ATP). Samples were then electrophoresed and blotted. The blots were then probed with either mouse monoclonal anti-PKR antibody (RiboGene, Inc.), rabbit polyclonal antibody against phosphorylated form of eIF-2α (Research Genetics, Inc), or goat polyclonal anti-eIF-2α antibody (Santa Cruz) using the ECL procedure as described (DuPont RENAISSANCE western blot chemiluminescence reagents). Anti-goat, anti-rabbit and anti-mouse antisera labeled with HRP were obtained from Jackson Immuno-Research Laboratories, Inc (Town, USA).

### In the presence of interferon

The PKR/dsRNA ratio is important for the initial activation of PKR. IFN-α, which has already was tested clinically against glioblastomas (Jereb et al. Acta Oncol (1994) 33:651-4) induces expression of PKR in U87MGΔEGFR cell lines (Figure 2). Indeed, treatment of transfected cells with IFNs, enhance expression of PKR expression. Figure 3 shows the results of increasing concentration of IFN-α on expression of PKR in U87MGAEGFR cell lines on Western blots.

Western Blot Analysis: Cells were seeded at a density of 1X10⁶ per 6cm diameter-petri dish and grown overnight. For determination of effect of IFN-α on PKR expression, cells were lysed in 300µl of Lysis Buffer 1 (10 % Glycerol, 0.05 M Tris-HCl pH-6.8, 5% β-mercaptoethanol, 3% SDS, 0.25 % Bromphenol Blue, 100 µg/ml PMSF, 1 mg/ml Aprotinin) and boiled. Equal amounts of proteins were electrophoresed through 10% SDS PAGE. (Figure 3). The separated proteins were then transferred to nitrocellulose membranes before being probed with rabbit polyclonal anti-PKR antibody (Santa Cruz) using ECL procedure as described (DuPont RENAISSANCE Western Blot Chemiluminescence reagents). Goat anti-rabbit and anti-mouse antiserums labeled with HRP were obtained from Jackson Immuno-Research Laboratories, Inc.

For determination of PKR activation, cells grown in the same conditions were transfected with either pΔEGFR-AS or pΔEGFR-S and treated with 500 U/ml of interferon (IFN-α) for 18 hours. PolyI-C was transfected 2 hours before lysis. Cells were then lysed with 500µl of Lysis Buffer 2 (50mM Tris-HCI pH 8.0, 150 MM NaCl, 0.02% Sodium Azide, 100µg/ml PMSF, 1 mg/ml Aprotinin, 1% Triton X-100) and the samples were centrifuged at 12,000xg for 5 min at 4°C to remove cell debris. PKR was immunoprecipitated with rabbit polyclonal anti-PKR antibody (Santa Cruz), electrophoresed and transferred to PVDF membranes (Millipore, Inc.). The blots were then probed with either mouse monoclonal antiphosphothreonine antibodies (Zymed Laboratories, Inc.), or polyclonal anti-PKR antibody using ECL procedure as described above. PKR concentration and phosphorylation were determined by scanning autoradiograms and measurement of optical density of each band by Macintosh NIH Image 1.61 software.

The above results are confirmed by transfecting cells with plasmids expressing β-galactoside in combination with pΔEGFR-AS and pΔEGFR-S plasmids. Cells were seeded into 24 well plates at a density of 10,000 per well and grown overnight. Cells were then transfected with 0.5µg of β-galactosidase expressing plasmid with a combination of 1.5µg pΔEGFR-AS or pΔEGFR-S plasmids and treated with 500 U/ml of IFN-α where indicated. β-galactosidase staining of the cells was then performed and stained cells were detected through a microscope connected to a digital camera. The results are shown in Figure 7.

### Measurement of PKR expression and cell growth using poly I-C

Poly I-C is a synthetic dsRNA molecule with indefinite length which activates PKR both *in vitro* and in intact cells (Hunter et al. (1975) Biol. Chem., 250:409-17; Clemens et al. (1975) Proc. Natl. Acad. Sci. USA 72:1286-90; Farrell et al. (1977) Cell, 11:187-200; Levin et al. (1978) Proc Natl Acad Sci USA, 75:1121-5; Levin et al. (1980) Proc Natl Acad Sci USA, 77 :832-6; Balachandran et al. (1998) EMBO J, 17:6888-902). As shown in Fig. 4, polyI-C has a strong effect on survival of both U87MGΔEGFR and U87MG cells leading to death of up to 84% of the cells. Treatment of the cells with IFN-α enhances the effect leading to 92% of cell death. Treatment of the U87MG cells with transfection reagent only (FuGene 6™) or transfection reagent in combination with IFN resulted in 38-39% cell death probably due to possible toxic effects of the reagent.

### Effect of dEGFR anti-sense RNA on induction of apoptosis

Since it was shown that activated PKR is a strong pro-apoptotic protein, induction of apoptosis can be tested in both uninfected and anti-sense RNA expressing vector- infected U87MG and U87MGΔEGFR cells. Changes in expression of pro-apoptotic proteins are shown to be increased by activation of PKR.

### Effect of transfection of PKR inhibitors on survival of the cells

To demonstrate that the death of the glioblastoma tells was caused by activation of PKR, it was shown that inhibitors of PKR could rescue the cells transfected with pΔEGFR-AS. Vaccinia PKR inhibitors (Davies et al. (1993) J. Virol; 67:1688-92) were selected for determining cell survival following PKR activation. In particular, the two vaccinia virus inhibitors of PKR - E3L and K3L, were used for the rescue experiment. The E3L gene of vaccinia virus encodes an inhibitor of the interferon-induced binding of double stranded RNA-dependent protein kinase to dsRNA. (Chang et al., (1992) Virology, 66: 1943-50.) The vaccinia virus K3L gene product potentiates translation by inhibiting double-stranded-RNA-activated protein kinase. This activity is thought to arise through the homology between K3L gene product and eIF-2α where the K3L gene product is an antagonist for binding with PKR (Davies et al. (1993) J. Virol., 67:1688-92).

Cells were seeded in 96 well plates at a density of 2000 cells per well and grown overnight. Cells were then transfected three times with 0.1µg of appropriate plasmids. Interval times between transfections were 24 hours. Methylene blue staining was conducted 24 hours after the last transfection and percent survival of the cells was calculated with respect to untreated cells. Figure 8 shows that co-transfection of plasmids encoding both inhibitors of PKR with pΔEGFR-AS, rescues the U87MGΔEGFR cells from death. Graphs A and B are results of the same experiment with B not showing effect of IFN-α.

Effects similar to the above with vaccinia virus proteins were observed when U87MGΔEGFR cells were infected with AS expressing vector and rescued with 2-aminopurine. The rescue by aminopurine further supports the involvement of PKR in cell death. Furthermore cotransfection of a plasmid encoding a transdominant negative mutant of PKR, PKRΔ6 with pΔEGFR-AS almost completely abrogated the effect of pΔEGFR-AS transfection alone.

### Effect of ΔEGFR anti-sense RNA on growth of U87MGΔEGFR cells injected to mouse

To test the effect of ΔEGFR anti-sense RNA on cancer growth in *in vivo* conditions, U87MGΔEGFR cells are injected into mouse and the multiplication rate of the neoplastic cells is measured. Anti-sense RNA expressing vector is then introduced into the mice and the mice are further treated with IFN-α. The control consists of a group of mice that do not receive cancer cells but are infected with an anti-sense RNA expressing vector to test for possible side effects.

### Example 6: PKR involvement in cell killing

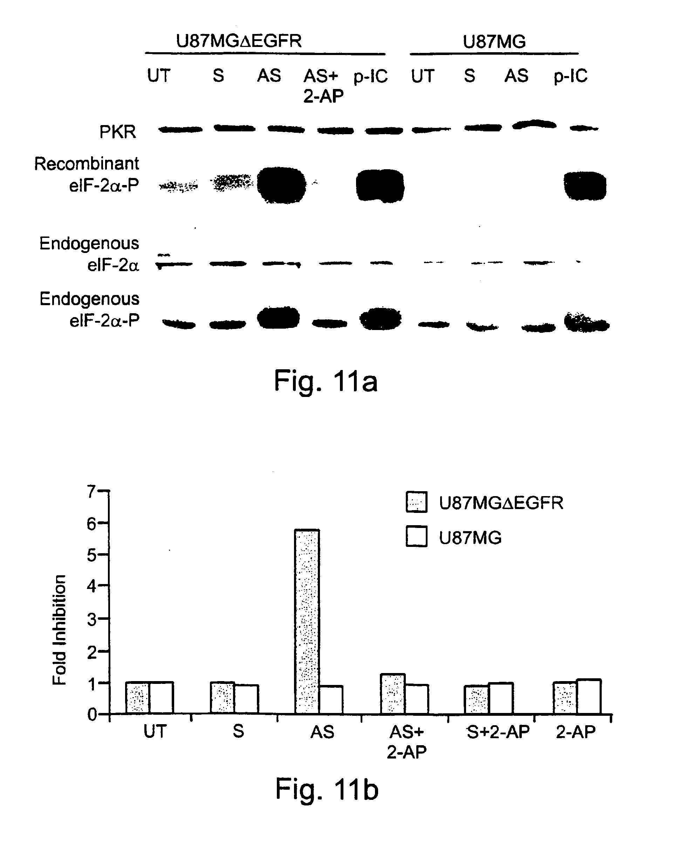

In order to examine PKR involvement in cell killing first the status of PKR activity was examined in the cells. In glioblastoma U87MG cells infected with anti-sense expressing vector PKR activation was identical either with sense infected or untreated cells (Fig. 10a). In contrast, infection of U87MGΔEGFR cells with AS RNA expressing vector led to strong PKR activation comparable with activation of PKR in cells transfected with the synthetic dsRNA molecule polyI-C. (Figure 11a) PKR activation was completely inhibited in cells treated with the PKR inhibitor 2-aminopurine (2-AP). Infection of U87MGΔEGFR cells with AS RNA expressing vector also stimulated phosphorylation of endogenous eIF-2α while in U87MG cells phosphorylation of eIF-2α remained at the same level.

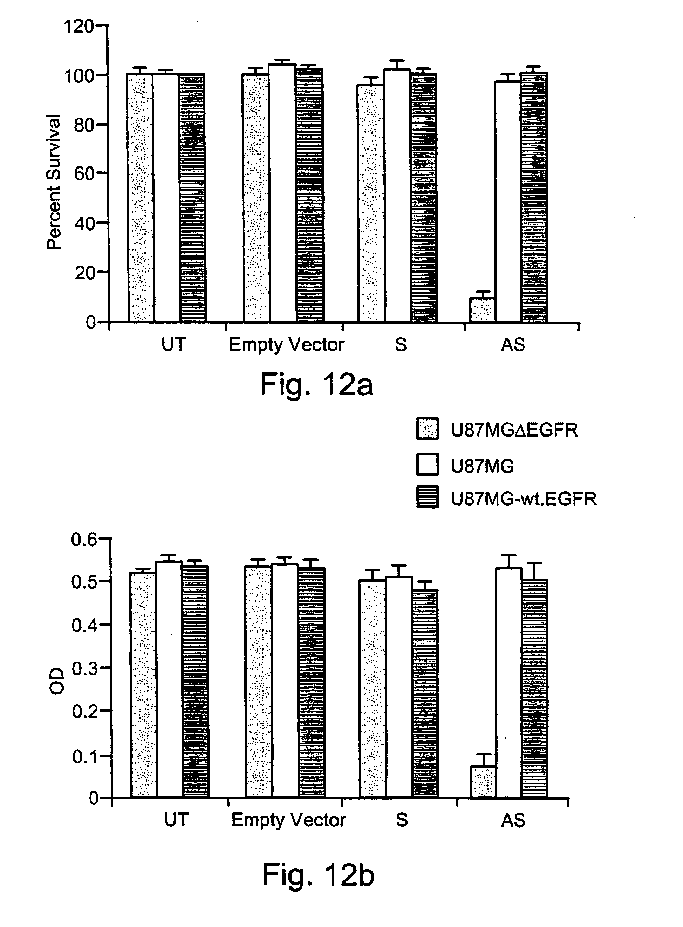

Next, whether the ΔEGRF-AS-induced activation of PKR does in fact lead to global inhibition of protein synthesis was examined. Infection of the U87MGΔEGFR cells with anti-sense expressing vector results in an almost 6-fold reduction in the total protein/total mRNA ratio. Elimination of this effect through the treatment of the AS infected cells with the 2-aminopurine, implies that PKR was directly involved in the inhibition of total translation. Treatment of the U87MGΔEGFR cells infected with AS expressing vector with 2-aminopurine rescued the cells, further supporting the involvement of PKR in cell death. Furthermore cotransfection of a plasmid encoding a transdominant negative mutant of PKR, PKRΔ6 (Koromilas et al. Science 257: 1685-9) with pΔEGFR-AS almost completely abrogated the effect of pΔEGFR-AS transfection alone (Figure 12). Transfection of plasmids encoding E3L and K3L, Vaccinia virus proteins that inhibit PKR, had similar effects.

### Example 7: Targeted cell killing in Lymphoma cells

Every t(14; 18) translocation has a unique sequence as a result of the different potential breakpoints on the two partner chromosomes and the random insertion of nucleotides at the fusion point ('N' region).

### Cell culture.

Lymphoma cell lines Nalm6: human pre-B-cell line (Hurwitz et al., 1979 Seminars in Oncology 25:447-460); Lam: human B-cell lymphoma which over-expresses *bcl-2* due to the translocation t(14;18); and Karpas299: T-cell lymphoma which is characterized by translocation t(2:5) resulting in the expression of the NPM-ALK fusion protein (Fischer et al., 1988 Blood 72:234-240) were used here. All lymphoma cell lines are grown in RPMI 1640 with 10% fetal calf serum (FCS) (Beit Haemek, Israel), 100µg/L streptomycin, 100,000 U/L penicillin. The HeLa, 3T3 L1 Tet-Off and 293T cell lines are grown in DMEM with 10% FCS, 100µg/L streptomycin, 100,000 U/L penicillin. The medium for 3T3 L1 Tet-Off is supplemented with 100µg/ml G418 and 2µg/ml doxycycline.

### Vectors.

*A.* The 77nt and 47nt long antisense and sense oligonucleotides corresponding to the sequences flanking the translocation regions t(14;18) *bcl-2*/*IgH* and t(2;5) NPM/ALK, respectively, were synthesized and cloned into XhoI and Nsil sites of the U6 expression vector. (He et al., 1998 J. Natl. Cancer Inst. 90: 1080-1087).
   The antisense and sense sequences for the t(2;5) were according to the translocation sequence published in the literature (Morris et al., 1994 Science 263: 1281-1284):
   *NPM*/*ALK*-antisense:
      SEQ ID No 11
      TCGAGGTGCTTCCGGCGGTACACTACTAAGTGCTGTCCACTAATGCA
   *NPM*/*ALK*-sense:
      SEQ ID No 12
      TCGAGTAGTGGACAGCACTTAGTAGTGTACCGCCGGAAGCACATGCA

   Since there are many potential breakpoints, the sequences of the antisense and sense for t(14;18) translocation were determined by nested PCR (see below):
   *Bcl-2*/*IgH-antisense:*
   *Bcl-2*/*IgH-sense:*
B. For lentiviral vector production the whole U6 cassette was excised by BamHI/EcoRI digestion, filled in with Klenow enzyme and inserted into the XhoI site (filled in) of the lentiviral vector pRRL-PGK-SIN (Deglon et al., 2000 Human Gene Therapy 11:179-190).

### Nested PCR.

Genomic DNA was purified from Lam and Nalm-6 cell lines as described (Edwards et al., 1991 Nucleic Acid Research 19: 1349). The first PCR was performed using the following set of primers:
SEQ ID No 15
   JH-5'ACCTGAGGAGACGGTGACCAGGGT3' and
SEQ ID No 16
   PA1- 5'AGTTATGGCCTATACACTATTTGT3'.

The second PCR utilized the product of the first PCR as a template, and the primers:
SEQ ID No 17
   PA2- 5'TTGTGAGCAAAGGTGATCGT3'
and
SEQ ID. No 18
   PA3-5'CAGGGTCCCTTGGCCCCAG3'

The conditions for the two PCR reactions were the same (100µM primers, 2.5mM dNTPs, 1.5mM MgCl2, 5 min at 94(C; 29 cycles of: 30 sec at 94(C, 30 sec at 55(C and 2 min at 86(C; 7 min at 72(C).

### IFNα treatment.

Cells were seeded onto 6-well plates at a density of 5x10⁵ cells/well and IFNα (500U/ml) was added. Cells were lysed after 20h using sample buffer (40% glycerol, 0.2M Tris HCl, pH 6.8, 20% β-Mercaptoethanol, 12% SDS, Bromo-Phenol Blue).

### Western blot analysis.

Equal amounts of proteins were loaded onto SDS-PAGE. The separated proteins were transferred to a nitrocellulose membrane, probed with the appropriate antibody, and visualized using ECL procedure. Secondary antibodies labeled with HRP were obtained from Jackson Immuno-Research Laboratories, Inc.

### PKR radioactive kinase assay.

The procedure was based on the literature (Patel and Sen, 1998) with certain changes. Cells were washed in cold-ice PBS and packed by centrifugation at 600g for 5 min (adherent cells were trypsinized before). Thereafter, they were lysed in lysis buffer (20mM Tris HCl, pH 7.5, 5mM MuCl₂, 50mM KCl, 400mM NaCl, 2mM DTT, 20% glycerol, 1% Triton X-100, 10µg/ml aprotinin, 2.4µg.ml AEBSF). The lysates were centrifuged at 10, 000g for 5 min and the supernatants were assayed for PKR activity. 500µg aliquots of total protein were immunoprecipitated using monoclonal PKR antibody (Ribogene) in high salt buffer (20mM Tris HCl, pH 7.5, 50mM KCI, 400mM NaCl, 1mM EDTA, 1mM DTT, 20% glycerol, 1% Triton X-100, 10µg/ml aprotinin, 2.4µg.ml AEBSF) at 4°C for 1 hr on a rotating wheel. 50µl of 20% protein A-Sepharose beads were then added and incubation was carried out for a further 1 h. Thereafter, the protein A-Sepharose beads were washed four times in high salt buffer and twice in activity buffer (20mM Tris HCl, pH 7.5, 2mM MgCl₂, 2mM MnCl₂, 50mM KCI, 20% glycerol, 1% Triton X-100, 10µg/ml aprotinin, 2.4µg.ml AEBSF). The PKR assay was performed in activity buffer to which were added the bead-bound PKR, 500ng purified eIF2α, 0.1mM ATP and 10Ci of [γ-³²P]ATP. The reaction proceeded at 30°C for 10 min. PolyI-C at various concentrations (0.1, 1 and 10µg/ml) was used to activate PKR. The reaction was halted by the addition of sample buffer (40% glycerol, 0.2M Tris HCl, pH 6.8, 20% β-Mercaptoethanol, 12% SDS, Bromo-Phenol Blue). The samples were boiled and separated by 10% SDS-PAGE. The separated proteins were transferred to a nitrocellulose membrane, which was exposed to a phosphorimager plate. The radioactive bands were detected in a phosphorimager (FUJIX, BAS 1000). The same membrane was also probed with PKR antibody (71/10, Ribogene, 1:5000).

### PKR non-radioactive kinase assay.

Cell lysates were prepared as described above. The reaction consisted of 50µg aliquots of total protein in 100µl of kinase reaction containing 20mM Tris-Hcl, pH 7.5, 50mM KCI, 2mM MuCl₂, 2mM MnCl₂, 5% glycerol, 10mM NaF, 100µM ATP, 10µg/ml aprotinin, 2.4µg.ml AEBSF in the presence of various concentration of pIC. The mixture was incubated on ice for 10 min to allow association of dsRNA with PKR. Then, 500ng purified eIF2α was added to the reaction and incubated at 30°C for 20 min. The reaction was halted by the addition of sample buffer. The samples were boiled and separated by 12% SDS-PAGE. The separated proteins were transferred to a nitrocellulose membrane. Phosphorylated substrate was detected by a specific antibody to the phosphorylated form of eIF2α (Research Genetics, 1:10,000). The same membrane was also probed with PKR antibody (71/10, Ribogene, 1:5000).

### Lentiviral vector production.

2X10⁶ 293T cells are seeded onto 10 cm plates. The pseudotyped HIV vectors are generated by transient cotransfection of a vector construct containing the GFP gene and the antisense or sense (pRRL-PGK-SIN, 20µg), with the VSV-G-expressing construct (pMD.G, 10µg) and the packaging construct (pCMVΔR8.91, 10µg), using FuGene6™ (Boehringer Mannheim). The medium is collected 48 hr after transfection, and various volumes of medium are used to infect the various cell lines.

### Cell infection.

2X10⁶ cells are seeded onto non-tissue culture 6-well plates (Falcon) coated with RetroNectin (Takara Shuzo Co, Ltd, Japan) according to the manufacturer's instructions. The medium containing the virus is added into the wells, and GFP expressing cells are visualised by fluorescence microscopy, 48 hr after infection. When antisense/sense-expressing vectors are used to infect the cells, they will be seeded on 96-well plates coated with RetroNectin.

### Measurement of global translation level.

Equal numbers of cells will be taken for extraction of total protein and total mRNA (PolyA Tract mRNA Isolation System III, Promega). Total protein concentration is determined by Coomassie blue staining, while total RNA concentration is determined by spectrophotometric analysis at 260/280nm. The ratio of total protein to total mRNA provides a standardized value for the amount of translation.

### Cell growth assay.

Cell growth will be determined by the microculture methylene blue assay (Ben Bassat et al., 1995).

### Apoptosis assays.

For FACS analysis, Cells will be stained with Annexin-V-FlOUS +PI according to the manufacturer's instruction (Boehringer Mannheim). For DNA fragmentation analysis, cells will be fix with 70% ethanol for 1h on ice, and then incubated with PI solution (50µg/ml PI, 0.1% triton X-100, 100µg/ml RNAse A, in PBS).

### Sequencing of the translocation junction t (14; 18) in Lam cells

In order to sequence the translocation junction in Lam cell line, a nested PCR reaction was performed on genomic DNA purified from Lam and Nalm-6 cells. As expected, no product was obtained using Nalm-6 DNA. Sequencing of the PCR product obtained from Lam DNA revealed the translocation junction between *bcl-2* and *IgH* (Fig. 1). The sequence for the translocation junction in Karpas299 cell line was taken from the literature (Morris et al., 1994).

### bcl-2/IgH: Translocation junction in LAM Cells

Bcl2 sequence
SEQ ID No 19
401 CTCCTTCCGC GGGGGCTTTC TCATGGCTGT CCTTCAGGGT CTTCCTGAAA

Sequence of the fusion point, in which random nucleotides are inserted during chromosome recombination.
SEQ ID No 20
451 TGCAGTGGTG CTTAC CCGGC GCCCCGTTAC CCCCTGTGTG CGAATGGGAT
IgH CDR3 region sequence
SEQ ID No 21
501 GAAAATACNG TATGGACGTC TGGGGCCAAG GGA

### Lentiviral infection of the Nalm6 and Karpas299 cell lines

The Nalm6, Lam and Karpas299 cell lines were infected with GFP encoding virus. Lentiviral vector production and infection were performed as described above. Two days after infection the cells expressing GFP were visualized and counted under the fluorescence microscope. The number of cells expressing GFP was divided by total cell number to yield the infection efficiency. When 0.5 ml (out of 8 ml) of medium containing virus was utilized for infection, 50% of the Nalm6 cells were found to be infected, whereas the infection efficiency of Karpas 299 was only 35%. These preliminary results show that genetic material can be introduced into these cell lines using lentiviral vectors. Efforts should however, be made to concentrate the virus in order to improve the yield of infection.

### PKR expression in Lam, Nalm-6 and Karpas 299 cell lines

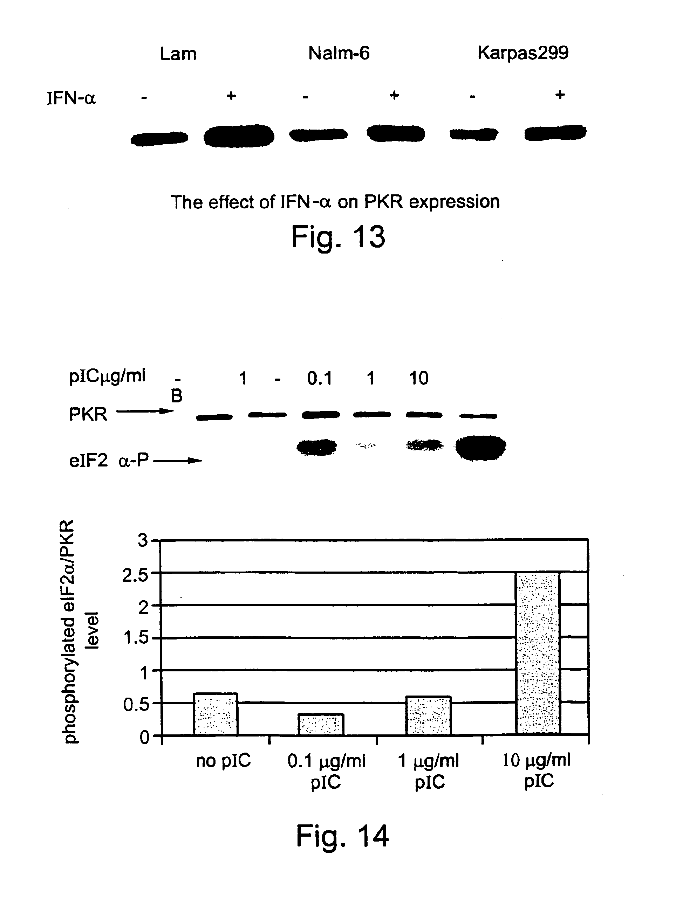

Cells were grown with or without IFN-α (500U/ml) for 20 h. Thereafter, they were lysed with sample buffer, and 30 µg of total protein was separated by 10% SDS-PAGE. The blot was incubated with anti-PKR (Ribogen). The results are shown in Fig 13. The results show that IFN-α induces *pkr* transcription, as expressed by elevated levels of PKR, in Lam, Nalm-6 and Karpas299 cell lines.

### PKR kinase assay

Activation of PKR was examined in Karpas299 cell line treated with IFNα, by a radioactive kinase assay, as described in "Materials and Methods". PKR levels detected in the various samples and eIF2α phosphorylation are shown in Fig 14A. Normalization of the Substrate phosphorylation to PKR level shows that PKR is activated by 10µg/ml of pI-C (Fig 14B).

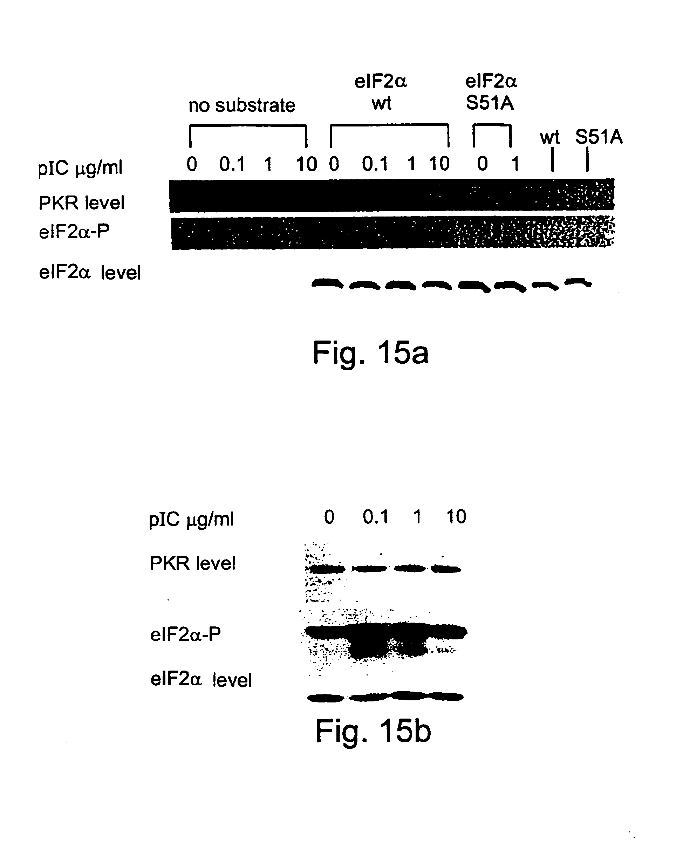

In order to simplify the assay two changes were made: 1) a specific antibody was used for the phosphorylated form of eIF2α instead of using radioactively labeled ATP. 2) The assay was performed on lysates instead of immunoprecipitants. A non-radioactive assay using lysates of Karpas299 treated with IFNα was performed as described above. For controls, the reaction was performed either without any substrate or with a mutant form of eIF2α that can not be phosphorylated (eIF2 αS51A). The results are shown in Fig.13. The phosphorylated form of eIF2α was detected by a specific antibody only in samples containing both lysate and wt substrate. The highest detection of phosphorylated eIF2α was found in a sample that was subjected to 1µg/ml of pI-C. No phosphorylated eIF2α was detected in samples that did not contain wt or mutant substrate. Also, no phosphorylated eIF2α was detected in the last two lanes that were loaded solely with the purified substrates, wt or mutant. The results show that specific activation of PKR can be achieved in lysates from Karpas299. Interestingly, when performing the assay on lysates from karpas 299 cells that were not treated with IFNα, activation of PKR was revealed in lower concentrations of pI-C (Fig.15). The need for higher concentration of pI-C in order to activate PKR may be due to IFNα treatment which causes elevation of PKR expression (Fig. 13).

## Claims

1. A method of *in-vitro* killing, or inhibiting growth of, a target cell expressing double stranded RNA dependent protein kinase, the method comprising introducing into the target cell at least one antisense RNA being capable of hybridizing with a transcribed RNA in the target cell to thereby form a double stranded RNA being at least 30 nucleotides in length,
wherein said at least one antisense RNA is selected from the group consisting of SEQ ID NO: 1, SEQ ID NO: 3, SEQ ID NO: 5, SEQ ID NO: 7, an RNA encoded by SEQ ID NO: 2, an RNA encoded by SEQ ID NO: 4, an RNA encoded by SEQ ID NO: 9, an RNA encoded by SEQ ID NO: 11 and an RNA encoded by SEQ ID NO: 13, thereby killing, or inhibiting growth of, the target cell.

2. A method of killing, or inhibiting growth of, a target cell in-vitro, the method comprising introducing into the target cell at least one antisense RNA being capable of hybridizing with a transcribed RNA in the target cell to thereby form a double stranded RNA being at least 30 nucleotides in length,
wherein the method further comprises administering to the target cell an interferon for activating, and/or elevating levels of, a double stranded RNA dependent protein kinase in the target cell, thereby killing, or inhibiting growth of, the target cell.

3. A method of in-vitro killing a target cell expressing a double stranded RNA dependent protein kinase and a transcript unique to the target cell , the method comprising introducing into the target cell at least one antisense RNA being capable of hybridizing with a nucleic acid sequence unique to the transcript in the target cell to thereby form a double stranded RNA being at least 30 nucleotides in length,
thereby activating the double stranded RNA dependent protein kinase in the target cell and killing the target cell.

4. A method of in-vitro killing a target cell expressing an mRNA sequence of Δ 2-7 EGFR and also a double stranded RNA dependent protein kinase, the method comprising introducing into the target cell an antisense RNA being capable of hybridizing with a contiguous RNA sequence which spans a deletion region of the Δ 2-7 EGFR, so as to form a double stranded RNA of 30-100 bp in the target cells, thereby activating the double stranded RNA dependent protein kinase in the target cells and killing the target cells.

5. A method according to claim 1, wherein the method further comprises administering to the target cell an interferon for activating, and/or elevating levels of, the double stranded RNA dependent protein kinase in the target cell.

6. A method according to claim 3, wherein the method further comprises administering to the target cell an interferon for activating, and/or elevating levels of, the kinase in the target cell.

7. A method according to claim 2, 3 or 4, wherein said at least one antisense RNA is selected from the group consisting of SEQ ID NO: 1, SEQ ID NO: 3, SEQ ID NO: 5, SEQ ID NO: 7, an RNA encoded by SEQ ID NO: 2, an RNA encoded by SEQ ID NO: 4, an RNA encoded by SEQ ID NO: 9, an RNA encoded by SEQ ID NO: 11 and an RNA encoded by SEQ ID NO: 13.

8. A method according to claim 1, 2 or 3, wherein said introducing said antisense RNA into the target cell comprises introducing into the target cell a nucleic acid encoding said antisense RNA in the target cell.

9. A method according to claim 9, wherein said introducing said nucleic acid into the target cell is effected using a delivery vehicle selected from the group consisting of a plasmid, a virus, a liposome, a naked DNA, a retrovirus, an adenovirus, a vaccinia virus, a herpesvirus, a DNA attached to a carrier and a lentivirus.

10. A method according to claim 1, 2 or 3, wherein the target cell is selected from the group consisting of a neoplastic cell, an autoimmune T-lymphocyte, an infectious agent, a smooth muscle cell in blood vessel plaque, a cell in psoriasis, a cell in keloid scarring, a neoplastic cell, a glioblastoma cell and a lymphoma cell.

11. Use of a nucleic acid in the manufacture of a medicament for treating a disorder **characterized by** proliferating cells expressing a double stranded RNA dependent protein kinase, the nucleic acid being an antisense RNA or encoding said antisense RNA in said proliferating cells, said antisense RNA being capable of hybridizing with a transcribed RNA in said proliferating cells to thereby form a double-stranded RNA being at least 30 nucleotides in length,
wherein said antisense RNA is selected from the group consisting of SEQ ID NO: 1, SEQ ID NO: 3, SEQ ID NO: 5, SEQ ID NO: 7, an RNA encoded by SEQ ID NO: 2, an RNA encoded by SEQ ID NO: 4, an RNA encoded by SEQ ID NO: 9, an RNA encoded by SEQ ID NO: 11 and an RNA encoded by SEQ ID NO: 13.

12. Use of a nucleic acid in the manufacture of a medicament for treating a disorder **characterized by** proliferating cells, the nucleic acid being an antisense RNA or encoding said antisense RNA in said proliferating cells, said antisense RNA being capable of hybridizing with a transcribed RNA in said proliferating cells to thereby form a double-stranded RNA being at least 30 nucleotides in length,
wherein the medicament comprises an interferon for activating, and/or elevating levels of, a double stranded RNA dependent protein kinase in said proliferating cells.

13. Use of a nucleic acid in the manufacture of a medicament for treating a disorder **characterized by** proliferating cells expressing Δ 2-7 EGFR and double stranded RNA dependent protein kinase, the nucleic acid being an antisense RNA or encoding said antisense RNA, said antisense RNA being capable of hybridizing with a contiguous RNA sequence which spans a deletion region of the Δ 2-7 EGFR so as to form a double stranded RNA of 30-100 bp in the proliferating cells.

14. The use according to claim 12, wherein said proliferating express a double stranded RNA dependent protein kinase.

15. The use according to claim 13, wherein said proliferating cells express said kinase.

16. The use according to claim 12, wherein the medicament comprises an interferon for activating, and/or elevating levels of, a double stranded RNA dependent protein kinase in said proliferating cells.

17. The use according to claim 14, wherein the medicament comprises an interferon for activating, and/or elevating levels of, said kinase in said proliferating cells.

18. The use according to claim 13 or 14, wherein said antisense RNA is selected from the group consisting of SEQ ID NO: 1, SEQ ID NO: 3, SEQ ID NO: 5, SEQ ID NO: 7, an RNA encoded by SEQ ID NO: 2, an RNA encoded by SEQ ID NO: 4, an RNA encoded by SEQ ID NO: 9, an RNA encoded by SEQ ID NO: 11 and an RNA encoded by SEQ ID NO: 13.

19. The use according to claim 12 or 13, wherein said proliferative disorder is selected from the group consisting of a neoplastic disease, psoriasis, a disorder which results from abnormal autoimmune T-lymphocytes, a disorder which includes vasculogenesis and a disorder which includes angiogenesis.

20. A pharmaceutical composition for treating a disorder **characterized by** proliferating cells expressing a double stranded RNA dependent protein kinase, the pharmaceutical composition comprising a physiologically acceptable carrier and a nucleic acid being an antisense RNA or being for expression of said antisense RNA in said proliferating cells, wherein the pharmaceutical composition comprises said nucleic acid in an amount effective for inhibiting growth of or killing the proliferating cells, said antisense RNA being capable of hybridizing with a transcribed RNA in said proliferating cells to thereby form a double-stranded RNA being at least 30 nucleotides in length,
wherein said antisense RNA is selected from the group consisting of SEQ ID NO: 1, SEQ ID NO: 3, SEQ ID NO: 5, SEQ ID NO: 7, an RNA encoded by SEQ ID NO: 2, an RNA encoded by SEQ ID NO: 4, an RNA encoded by SEQ ID NO: 9, an RNA encoded by SEQ ID NO: 11 and an RNA encoded by SEQ ID NO: 13.

21. A pharmaceutical composition for treating a disorder **characterized by** proliferating cells, the pharmaceutical composition comprising a physiologically acceptable carrier and a nucleic acid being an antisense RNA or being for expression of said antisense RNA in said proliferating cells, wherein the pharmaceutical composition comprises said nucleic acid in an amount effective for inhibiting growth of or killing the proliferating cells, said antisense RNA being capable of hybridizing with a transcribed RNA in said proliferating cells to thereby form a double-stranded RNA being at least 30 nucleotides in length,
wherein the pharmaceutical composition further comprises an interferon for activating, and/or elevating levels of, a double stranded RNA dependent protein kinase in the proliferating cells.

22. A pharmaceutical composition for treating a disorder **characterized by** proliferating cells expressing a double stranded RNA dependent protein kinase, the pharmaceutical composition comprising a physiologically acceptable carrier and a nucleic acid being an antisense molecule or encoding said antisense molecule, said antisense molecule being capable of hybridizing with a contiguous RNA sequence which spans a deletion region of Δ 2-7 EGFR so as to form a double stranded RNA of 30-100 bp in proliferating cells expressing said Δ 2-7 EGFR.

23. The pharmaceutical composition according to claim 22, wherein said proliferating cells express said kinase.

24. The pharmaceutical composition according to claim 21, wherein the pharmaceutical composition further comprises an interferon for activating, and/or elevating levels of, a double stranded RNA dependent protein kinase in said proliferating cells

25. The pharmaceutical composition according to claim 23, wherein the pharmaceutical composition further comprises an interferon for activating, and/or elevating levels of, said kinase in said proliferating cells

26. The pharmaceutical composition according to claim 22 or 23, wherein said antisense RNA is selected from the group consisting of SEQ ID NO: 1, SEQ ID NO: 3, SEQ ID NO: 5, SEQ ID NO: 7, an RNA encoded by SEQ ID NO: 2, an RNA encoded by SEQ ID NO: 4, an RNA encoded by SEQ ID NO: 9, an RNA encoded by SEQ ID NO: 11 and an RNA encoded by SEQ ID NO: 13.

27. A composition comprising an antisense RNA, said antisense RNA being selected from the group consisting of SEQ ID NO: 1, SEQ ID NO: 3, SEQ ID NO: 5, SEQ ID NO: 7, an RNA encoded by SEQ ID NO: 2, an RNA encoded by SEQ ID NO: 4, an RNA encoded by SEQ ID NO: 9, an RNA encoded by SEQ ID NO: 11 and an RNA encoded by SEQ ID NO: 13.

28. A nucleic acid comprising a nucleotide sequence encoding an antisense RNA, said antisense RNA being selected from the group consisting of SEQ ID NO: 1, SEQ ID NO: 3, SEQ ID NO: 5, SEQ ID NO: 7, an RNA encoded by SEQ ID NO: 2, an RNA encoded by SEQ ID NO: 4, an RNA encoded by SEQ ID NO: 9, an RNA encoded by SEQ ID NO: 11 and an RNA encoded by SEQ ID NO: 13.

29. A vector comprising a nucleotide sequence encoding an antisense RNA, said antisense RNA being selected from the group consisting of SEQ ID NO: 1, SEQ ID NO: 3, SEQ ID NO: 5, SEQ ID NO: 7, an RNA encoded by SEQ ID NO: 2, an RNA encoded by SEQ ID NO: 4, an RNA encoded by SEQ ID NO: 9, an RNA encoded by SEQ ID NO: 11 and an RNA encoded by SEQ ID NO: 13.

30. The vector according to claim 30, wherein the vector is a retroviral vector.

31. The vector according to claim 30, wherein said retroviral vector is a lentivirus.

32. A cell transfected with a vector according to claim 30.

33. The cell according to claim 33, wherein the cell is stably transfected.

## Patentansprüche

1. Verfahren der *in*-*vitro*-Vernichtung oder der Wachstumshemmung einer Zielzelle, die eine zweisträngige RNA-abhängige Proteinkinase exprimiert, wobei das Verfahren das Einführen wenigstens eines Antisense-RNAs in die Zielzelle umfasst, das in der Lage ist, sich in der Zielzelle mit einem transkribierten RNA zu hybridisieren um **dadurch** ein zweisträngiges RNA mit einer Länge von wenigstens 30 Nukleotiden zu bilden,
wobei das genannte wenigstens eine Antisense-RNA ausgewählt ist aus der Gruppe bestehend aus der SEQ ID Nr.: 1, SEQ ID Nr.: 3, SEQ ID Nr.: 5, SEQ ID Nr.: 7, einem aus der SEQ ID Nr.: 2 kodierten RNA, einem aus der SEQ ID Nr.: 4 kodierten RNA, einem aus der SEQ ID Nr.: 9 kodierten RNA, einem aus der SEQ ID Nr.: 11 kodierten RNA und einem aus der SEQ ID Nr.: 13 kodierten RNA, wodurch es zur Vernichtung oder der Wachstumshemmung der Zielzelle kommt.

2. Verfahren der Vernichtung oder der Wachstumshemmung einer Zielzelle in-vitro,
wobei das Verfahren das Einführen wenigstens eines Antisense-RNA in die Zielzelle umfasst, das in der Lage ist, sich in der Zielzelle mit einem transkribierten RNA zu hybridisieren um **dadurch** ein zweisträngiges RNA mit einer Länge von wenigstens 30 Nukleotiden zu bilden,
wobei das Verfahren ferner das Verabreichen von Interferon zur Aktivierung und/oder Erhöhung der Werte einer zweisträngigen RNA-abhängigen Proteinkinase in die Zielzelle umfasst, wodurch es zur Vernichtung oder der Wachstumshemmung der Zielzelle kommt.

3. Verfahren der in-vitro-Vernichtung einer Zielzelle, die eine zweisträngige RNA-abhängige Proteinkinase und ein für Zielzelle eindeutiges Transkript exprimiert, wobei das Verfahren das Einführen wenigstens eines Antisense-RNA in die Zielzelle umfasst, das in der Lage ist sich mit einer Nukleinsäuresequenz zu hybridisieren, die für das Transkript in der Zielzelle eindeutig ist, um **dadurch** ein zweisträngiges RNA mit einer Länge von wenigstens 30 Nukleotiden zu bilden,
um **dadurch** die zweisträngige RNA-abhängige Proteinkinase in der Zielzelle zu aktivieren und die Zielzelle zu vernichten.

4. Verfahren der in-vitro-Vernichtung einer Zielzelle, die eine mRNA-Sequenz von Δ 2-7 EGFR und ebenfalls eine zweisträngige RNA-abhängige Proteinkinase umfasst,
wobei das Verfahren das Einführen eines Antisense-RNA in die Zielzelle umfasst, die in der Lage ist, sich mit einer angrenzenden RNA-Sequenz zu hybridisieren, die sich derart über einen Deletionsbereich des Δ 2-7 EGFR erstreckt, um ein zweisträngiges RNA von 30-100 bp in den Zielzellen zu bilden, um **dadurch** die zweisträngige RNA-abhängige Proteinkinase in den Zielzellen zu aktivieren und die Zielzellen zu vernichten.

5. Verfahren nach Anspruch 1, wobei das Verfahren ferner das Verabreichen von Interferon an die Zielzelle zum Aktivieren und/oder Erhöhen der Werte der zweisträngigen RNA-abhängigen Proteinkinase in der Zielzelle umfasst.

6. Verfahren nach Anspruch 3, wobei das Verfahren ferner das Verabreichen von Interferon an die Zielzelle zum Aktivieren und/oder Erhöhen der Werte der Kinase in der Zielzelle umfasst.

7. Verfahren nach Anspruch 2, 3 oder 4, wobei das genannte wenigstens eine Antisense-RNA ausgewählt ist aus der Gruppe bestehend aus der SEQ ID Nr.: 1, SEQ ID Nr.: 3, SEQ ID Nr.: 5, SEQ ID Nr.: 7, einem durch SEQ ID Nr.: 2 kodierten RNA, einem durch SEQ ID Nr.: 4 kodierten RNA, einem durch SEQ ID Nr.: 9 kodierten RNA, einem durch SEQ ID Nr.: 11 kodierten RNA und einem durch SEQ ID Nr.: 13 kodierten RNA.

8. Verfahren nach Anspruch 1, 2 oder 3, wobei das genannte Einführen des genannten Antisense-RNA in die Zielzelle das Einführen in die Zielzelle einer Nukleinsäure umfasst, die das genannte Antisense-RNA in die Zielzelle kodiert.

9. Verfahren nach Anspruch 9, wobei das genannte Einführen der genannten Nukleinsäure in die Zielzelle durch Verwendung eines Abgabeträgerstoffs durchgeführt wird, das ausgewählt ist aus der Gruppe bestehend aus einem Plasmid, einem Virus, einem Liposom, einer nackten DNA, einem Retrovirus, einem Adenovirus, einem vaccinia Virus, einem Herpesvirus, einem mit einem Träger und einem Lentivirus verbundenen DNA.

10. Verfahren nach Anspruch 1, 2 oder 3, wobei die Zielzelle ausgewählt ist aus der Gruppe bestehend aus einer neoplastischen Zelle, einem Autoimmun-T-Lymphozyten, einem Infektionserreger, einer glatten Muskelzelle in Blutgefässplaque, einer Zelle in Psoriasis, einer Zelle in einem Narbenkulus, einer neoplastischen Zelle, einer Glioblastomzelle und einer Lymphomzelle.

11. Verwendung einer Nukleinsäure bei der Herstellung eines Medikaments zur Behandlung einer Krankheit, die **dadurch gekennzeichnet ist**, eine zweisträngige RNA-abhängige Proteinkinase exprimierende Zellen zu vermehren, wobei die Nukleinsäure ein Antisense-RNA ist oder das genannte Antisense-RNA in den genannten vermehrten Zellen kodiert, wobei das genannte Antisense-RNA in der Lage ist, sich mit einem transkribierten RNA in den genannten vermehrten Zellen zu hybridisieren, um **dadurch** ein zweisträngiges RNA mit einer Länge von wenigstens 30 Nukleotiden zu bilden,
wobei das genannte Antisense-RNA ausgewählt ist aus der Gruppe bestehend aus der SEQ ID Nr.: 1, SEQ ID Nr.: 3, SEQ ID Nr.: 5, SEQ ID Nr.: 7, einem durch SEQ ID Nr.: 2 kodierten RNA, einem durch SEQ ID Nr.: 4 kodierten RNA, einem durch SEQ ID Nr.: 9 kodierten RNA, einem durch SEQ ID Nr.: 11 kodierten RNA und einem durch SEQ ID Nr.: 13 kodierten RNA.

12. Verwendung einer Nukleinsäure bei der Herstellung eines Medikaments zur Behandlung einer Krankheit, die **gekennzeichnet ist durch** das Vermehren von Zellen,
wobei die Nukleinsäure ein Antisense-RNA ist oder das genannte Antisense-RNA in den genannten vermehrten Zellen kodiert, wobei das genannte Antisense-RNA in der Lage ist, sich mit einem transkribierten RNA in den genannten vermehrten Zellen zu hybridisieren, um **dadurch** ein zweisträngiges RNA mit einer Länge von wenigstens 30 Nukleotiden zu bilden,
wobei das Medikament Interferon zum Aktivieren und/oder Erhöhen der Werte einer zweisträngigen RNA-abhängigen Proteinkinase in den genannten vermehrten Zellen umfasst.

13. Verwendung einer Nukleinsäure bei der Herstellung eines Medikaments zur Behandlung einer Krankheit, die **gekennzeichnet ist durch** das Vermehren von Zellen, die Δ 2-7 EGRF und eine zweisträngige RNA-abhängige Proteinkinase exprimieren,
wobei die Nukleinsäure ein Antisense-RNA ist oder das genannte Antisense-RNA kodiert, wobei das genannte Antisense-RNA in der Lage ist, sich mit einer angrenzenden RNA-Sequenz zu hybridisieren, die sich derart über einen Deletionsbereich des Δ 2-7 EGFR erstreckt, um ein zweisträngiges RNA aus 30 - 100 bp in den vermehrten Zellen zu bilden.

14. Verwendung nach Anspruch 12, wobei die genannten vermehrten Zellen eine zweisträngige RNA-abhängige Proteinkinase exprimieren.

15. Verwendung nach Anspruch 13, wobei die genannten vermehrten Zellen die genannte Kinase exprimieren.

16. Verwendung nach Anspruch 12, wobei das Medikament Interferon zum Aktivieren und/oder Erhöhen der Werte einer zweisträngigen RNA-abhängigen Proteinkinase in den genannten vermehrten Zellen umfasst.

17. Verwendung nach Anspruch 14, wobei das Medikament Interferon zum Aktivieren und/oder Erhöhen der Werte der genannten Kinase in den genannten vermehrten Zellen umfasst.

18. Verwendung nach Anspruch 13 oder 14, wobei das genannte Antisense-RNA ausgewählt ist aus der Gruppe bestehend aus der SEQ ID Nr.: 1, SEQ ID Nr.: 3, SEQ ID Nr.: 5, SEQ ID Nr.: 7, einem durch SEQ ID Nr.: 2 kodierten RNA, einem durch SEQ ID Nr.: 4 kodierten RNA, einem durch SEQ ID Nr.: 9 kodierten RNA, einem durch SEQ ID Nr.: 11 kodierten RNA und einem SEQ ID Nr.: 13 kodierten RNA.

19. Verwendung nach Anspruch 12 oder 13, wobei die genannte Proliferationskrankheit ausgewählt ist aus der Gruppe bestehend aus einer neoplastischen Krankheit, Psoriasis, einer durch abnormale Autoimmun-T-Lymphozyten entstehenden Krankheit, einer Krankheit, die Vaskulogenese und einer Krankheit, die Angiogenese beinhaltet.

20. Pharmazeutische Zusammensetzung zur Behandlung einer Krankheit, die durch vermehrte Zellen **gekennzeichnet** ist, die eine zweisträngige RNA-abhängige Proteinkinase exprimieren, wobei die pharmazeutische Zusammensetzung einen physiologisch akzeptablen Träger und eine Nukleinsäure umfasst, die ein Antisense-RNA ist oder zur Expression des genannte Antisense-RNA in den genannten vermehrten Zellen bestimmt ist, wobei die pharmazeutische Zusammensetzung die genannte Nukleinsäure in einer Menge umfasst, die zur Wachstumshemmung oder zur Vernichtung der vermehrten Zellen wirksam ist, wobei das Antisense-RNA in der Lage ist, sich mit einem transkribierten RNA in den genannten vermehrten Zellen zu hybridisieren, um **dadurch** ein zweisträngiges RNA mit einer Länge von wenigstens 30 Nukleotiden zu bilden,
wobei das genannte Antisense-RNA ausgewählt ist aus der Gruppe bestehend aus der SEQ ID Nr.: 1, SEQ ID Nr.: 3, SEQ ID Nr.: 5, SEQ ID Nr.: 7, einem durch SEQ ID Nr.: 2 kodierten RNA, einem durch SEQ ID Nr.: 4 kodierten RNA, einem durch SEQ ID Nr.: 9 kodierten RNA, einem durch SEQ ID Nr.: 11 kodierten RNA und einem durch SEQ ID Nr.: 13 kodierten RNA.

21. Pharmazeutische Zusammensetzung zur Behandlung einer Krankheit, die **gekennzeichnet ist durch** vermehrte Zellen, wobei die pharmazeutische Zusammensetzung einen physiologisch akzeptablen Träger und eine Nukleinsäure umfasst, die ein Antisense-RNA ist oder die zur Expression des genannten Antisense-RNA in den genannten vermehrten Zellen bestimmt ist, wobei die pharmazeutische Zusammensetzung eine Nukleinsäure in einer Menge umfasst, die zur Wachstumshemmung oder Vernichtung der vermehrten Zellen wirksam ist, wobei das genannte Antisense-RNA in der Lage ist, sich mit einem transkribierten RNA in den genannten vermehrten Zellen zu hybridisieren, um **dadurch** ein zweisträngiges RNA mit einer Länge von wenigstens 30 Nukleotiden zu bilden,
wobei die pharmazeutische Zusammensetzung ferner Interferon zur Aktivierung und/oder Erhöhung der Werte einer zweisträngigen RNA-abhängigen Proteinkinase in den vermehrten Zellen umfasst.

22. Pharmazeutische Zusammensetzung zur Behandlung einer Krankheit, die **gekennzeichnet ist durch** vermehrte Zellen, die eine zweisträngige RNA-abhängige Proteinkinase exprimieren, wobei die pharmazeutische Zusammensetzung einen physiologisch akzeptablen Träger und eine Nukleinsäure umfasst, die ein Antisense-Molekül ist oder das genannte Antisense-Molekül kodiert, wobei das genannte Antisense-Molekül in der Lage ist, sich mit einer angrenzenden RNA-Sequenz zu hybridisieren, die sich derart über einen Deletionsbereich von Δ 2-7 EGRF erstreckt, um ein zweisträngiges RNA von 30 - 100 bp in den vermehrten Zellen zu erstrecken, die das genannte Δ 2-7 EGRF exprimieren.

23. Pharmazeutische Zusammensetzung nach Anspruch 22, wobei die genannten vermehrten Zellen die genannte Kinase exprimieren.

24. Pharmazeutische Zusammensetzung nach Anspruch 21, wobei die pharmazeutische Zusammensetzung ferner Interferon zum Aktivieren und/oder Erhöhen der Werte einer zweisträngigen RNA-abhängigen Proteinkinase in den genannten vermehrten Zellen umfasst.

25. Pharmazeutische Zusammensetzung nach Anspruch 23, wobei die pharmazeutische Zusammensetzung ferner Interferon zum Aktivieren und/oder Erhöhen der Werte der genannten Kinase in den genannten vermehrten Zellen umfasst.

26. Pharmazeutische Zusammensetzung nach Anspruch 22 oder 23, wobei das genannte Antisense-RNA ausgewählt ist aus der Gruppe bestehend aus der SEQ ID Nr.: 1, SEQ ID Nr.: 3, SEQ ID Nr.: 5, SEQ ID Nr.: 7, einem durch SEQ ID Nr.: 2 kodierten RNA, einem durch SEQ ID Nr.: 4 kodierten RNA, einem durch SEQ ID Nr.: 9 kodierten RNA, einem durch SEQ ID Nr.: 11 kodierten RNA und einem durch SEQ ID Nr.: 13 kodierten RNA.

27. Zusammensetzung umfassend ein Antisense-RNA, wobei das Antisense-RNA ausgewählt ist aus der Gruppe bestehend aus der SEQ ID Nr.: 1, SEQ ID Nr.: 3, SEQ ID Nr.: 5, SEQ ID Nr.: 7, einem durch SEQ ID Nr.: 2 kodierten RNA, einem durch SEQ ID Nr.: 4 kodierten RNA, einem durch SEQ ID Nr.: 9 kodierten RNA, einem durch SEQ ID Nr.: 11 kodierten RNA und einem durch SEQ ID Nr.: 13 kodierten RNA.

28. Nukleinsäure umfassend eine Nukleotidsequenz, die ein Antisense-RNA kodiert,
wobei das genannte Antisense-RNA ausgewählt ist aus der Gruppe bestehend aus der SEQ ID Nr.: 1, SEQ ID Nr.: 3, SEQ ID Nr.: 5, SEQ ID Nr.: 7, einem durch SEQ ID Nr.: 2 kodierten RNA, einem durch SEQ ID Nr.: 4 kodierten RNA, einem durch SEQ ID Nr.: 9 kodierten RNA, einem durch SEQ ID Nr.: 11 kodierten RNA und einem durch SEQ ID Nr.: 13 kodierten RNA.

29. Vektor umfassend eine Nukleotidsequenz, die ein Antisense-RNA kodiert, wobei das genannte Antisense-RNA ausgewählt ist aus der Gruppe bestehend aus der SEQ ID Nr.: 1, SEQ ID Nr.: 3, SEQ ID Nr.: 5, SEQ ID Nr.: 7, einem durch SEQ ID Nr.: 2 kodierten RNA, einem durch SEQ ID Nr.: 4 kodierten RNA, einem durch SEQ ID Nr.: 9 kodierten RNA, einem durch SEQ ID Nr.: 11 kodierten RNA und einem durch SEQ ID Nr.: 13 kodierten RNA.

30. Vektor nach Anspruch 30, wobei der Vektor ein Retrovirus-Vektor ist.

31. Vektor nach Anspruch 30, wobei der genannte Retrovirus-Vektor ein Lentivirus ist.

32. Mit einem Vektor transfizierte Zelle nach Anspruch 30.

33. Zelle nach Anspruch 32, wobei die Zelle stabil transfiziert ist.

## Revendications

1. Procédé *in vitro* pour tuer ou inhiber la croissance d'une cellule cible exprimant une protéine kinase dépendante de l'ARN double brin, le procédé consistant à introduire dans la cellule cible au moins un ARN antisens capable de s'hybrider avec un ARN transcrit dans la cellule cible pour ainsi former un ARN double brin ayant une longueur d'au moins 30 nucléotides,
où ledit au moins un ARN antisens est sélectionné dans le groupe consistant en SEQ ID NO: 1, SEQ ID NO: 3, SEQ ID NO: 5, SEQ ID NO: 7, un ARN codé par SEQ ID NO: 2, un ARN codé par SEQ ID NO: 4, un ARN codé par SEQ ID NO: 9, un ARN codé par SEQ ID NO: 11 et un ARN codé par SEQ ID NO: 13, ce qui ainsi tue ou inhibe la croissance de la cellule cible.

2. Procédé pour tuer ou inhiber la croissance d'une cellule cible in vitro, le procédé consistant à introduire dans la cellule cible au moins un ARN antisens qui est capable de s'hybrider avec un ARN transcrit dans la cellule cible pour ainsi former un ARN double brin ayant une longueur d'au moins 30 nucléotides,
où le procédé consiste en outre à administrer à la cellule cible un interféron pour activer, et/ou augmenter les taux d'une protéine kinase dépendante de l'ARN double brin dans la cellule cible, ce qui ainsi tue ou inhibe la croissance de la cellule cible.

3. Procédé pour tuer in vitro une cellule cible exprimant une protéine kinase dépendante de l'ARN double brin et un transcrit unique à la cellule cible, le procédé consistant à introduire dans la cellule cible au moins un ARN antisens qui est capable de s'hybrider avec une séquence d'acide nucléique unique au transcrit dans la cellule cible pour ainsi former un ARN double brin ayant une longueur d'au moins 30 nucléotides,
ce qui active ainsi la protéine kinase dépendante de l'ARN double brin dans la cellule cible et tue la cellule cible.

4. Procédé pour tuer *in vitro* une cellule cible exprimant une séquence d'ARNm de Δ 2-7 EGFR et également une protéine kinase dépendante de l'ARN double brin, le procédé consistant à introduire dans la cellule cible un ARN antisens qui est capable de s'hybrider à une séquence d'ARN contiguë qui englobe une région de délétion du Δ 2-7 EGFR, de sorte à former un ARN double brin de 30-100 pb dans les cellules cibles, ce qui active ainsi la protéine kinase dépendante de l'ARN double brin dans les cellules cibles et tue les cellules cibles.

5. Procédé selon la revendication 1, où le procédé consiste en outre à administrer à la cellule cible un interféron pour activer, et/ou augmenter les taux de la protéine kinase dépendante de l'ARN double brin dans la cellule cible.

6. Procédé selon la revendication 3, où le procédé consiste en outre à administrer à la cellule cible un interféron pour activer, et/ou augmenter les taux de la kinase dans la cellule cible.

7. Procédé selon la revendication 2, 3 ou 4, où ledit au moins un ARN antisens est sélectionné dans le groupe consistant en SEQ ID NO: 1, SEQ ID NO: 3, SEQ ID NO: 5, SEQ ID NO: 7, un ARN codé par SEQ ID NO: 2, un ARN codé par SEQ ID NO: 4, un ARN codé par SEQ ID NO: 9, un ARN codé par SEQ ID NO: 11 et un ARN codé par SEQ ID NO: 13.

8. Procédé selon la revendication 1, 2 ou 3, où ladite introduction dudit ARN antisens dans la cellule cible consiste à introduire dans la cellule cible un acide nucléique codant pour ledit ARN antisens dans la cellule cible.

9. Procédé selon la revendication 9, où ladite introduction dudit acide nucléique dans la cellule cible est réalisée en utilisant un véhicule de délivrance sélectionné dans le groupe consistant en un plasmide, un virus, un liposome, un ADN nu, un rétrovirus, un adénovirus, un virus de la vaccine, un herpèsvirus, un ADN attaché à un transporteur et un lentivirus.

10. Procédé selon la revendication 1, 2 ou 3, où la cellule cible est sélectionnée dans le groupe consistant en une cellule néoplasique, un lymphocyte T auto-immun, un agent infectieux, une cellule musculaire lisse dans une plaque de vaisseau sanguin, une cellule dans un psoriasis, une cellule dans une cicatrice chéloïde, une cellule néoplasique, une cellule de glioblastome et une cellule de lymphome.

11. Utilisation d'un acide nucléique dans la fabrication d'un médicament pour traiter un trouble **caractérisé par** des cellules proliférantes exprimant une protéine kinase dépendante de l'ARN double brin, l'acide nucléique étant un ARN antisens ou codant pour ledit ARN antisens dans lesdites cellules proliférantes, ledit ARN antisens étant capable de s'hybrider avec ARN transcrit dans lesdites cellules proliférantes pour ainsi former un ARN double brin ayant une longueur d'au moins 30 nucléotides,
où ledit ARN antisens est sélectionné dans le groupe consistant en SEQ ID NO: 1, SEQ ID NO: 3, SEQ ID NO: 5, SEQ ID NO: 7, un ARN codé par SEQ ID NO: 2, un ARN codé par SEQ ID NO: 4, un ARN codé par SEQ ID NO: 9, un ARN codé par SEQ ID NO: 11 et un ARN codé par SEQ ID NO: 13.

12. Utilisation d'un acide nucléique dans la fabrication d'un médicament pour traiter un trouble **caractérisé par** des cellules proliférantes, l'acide nucléique étant un ARN antisens ou codant pour ledit ARN antisens dans lesdites cellules proliférantes, ledit ARN antisens étant capable de s'hybrider avec un ARN transcrit dans lesdites cellules proliférantes pour ainsi former un ARN double brin ayant une longueur d'au moins 30 nucléotides,
où le médicament comprend un interféron pour activer, et/ou augmenter des taux d'une protéine kinase dépendante de l'ARN double brin dans lesdites cellules proliférantes.

13. Utilisation d'un acide nucléique dans la fabrication d'un médicament pour traiter un trouble **caractérisé par** des cellules proliférantes exprimant un Δ 2-7 EGFR et une protéine kinase dépendante de l'ARN double brin, l'acide nucléique étant un ARN antisens ou codant pour ledit ARN antisens, ledit ARN antisens étant capable de s'hybrider avec une séquence d'ARN contiguë qui englobe une région de délétion du Δ 2-7 EGFR de sorte à former un ARN double brin de 30-100 pb dans les cellules proliférantes.

14. Utilisation selon la revendication 12, où lesdites cellules proliférantes expriment une protéine kinase dépendante de l'ARN double brin.

15. Utilisation selon la revendication 13, où lesdites cellules proliférantes expriment ladite kinase.

16. Utilisation selon la revendication 12, où le médicament comprend un interféron pour activer, et/ou augmenter les taux d'une protéine kinase dépendante de l'ARN double brin dans lesdites cellules proliférantes.

17. Utilisation selon la revendication 14, où le médicament comprend un interféron pour activer, et/ou élever les taux de ladite kinase dans lesdites cellules proliférantes.

18. Utilisation selon la revendication 13 ou 14, où ledit ARN antisens est sélectionné dans le groupe consistant en SEQ ID NO: 1, SEQ ID NO: 3, SEQ ID NO: 5, SEQ ID NO: 7, un ARN codé par SEQ ID NO: 2, un ARN codé par SEQ ID NO: 4, un ARN codé par SEQ ID NO: 9, un ARN codé par SEQ ID NO: 11 et un ARN codé par SEQ ID NO: 13.

19. Utilisation selon la revendication 12 ou 13, où ledit trouble prolifératif est sélectionné dans le groupe consistant en une maladie néoplasique, un psoriasis, un trouble résultant de lymphocytes T auto-immuns anormaux, un trouble qui comprend la vasculogenèse et un trouble qui comprend l'angiogenèse.

20. Composition pharmaceutique pour traiter un trouble **caractérisé par** des cellules proliférantes exprimant une protéine kinase dépendante de l'ARN double brin, la composition pharmaceutique comprenant un véhicule physiologiquement acceptable et un acide nucléique qui est un ARN antisens ou qui est pour l'expression dudit ARN antisens dans lesdites cellules proliférantes, où la composition pharmaceutique comprend ledit acide nucléique en une quantité efficace pour tuer ou inhiber la croissance des cellules proliférantes, ledit ARN antisens étant capable de s'hybrider avec un ARN transcrit dans lesdites cellules proliférantes pour ainsi former un ARN double brin ayant une longueur d'au moins 30 nucléotides,
où ledit ARN antisens est sélectionné dans le groupe consistant en SEQ ID NO: 1, SEQ ID NO: 3, SEQ ID NO: 5, SEQ ID NO: 7, un ARN codé par SEQ ID NO: 2, un ARN codé par SEQ ID NO: 4, un ARN codé par SEQ ID NO: 9, un ARN codé par SEQ ID NO: 11 et un ARN codé par SEQ ID NO: 13.

21. Composition pharmaceutique pour traiter un trouble **caractérisé par** des cellules proliférantes, la composition pharmaceutique comprenant un véhicule physiologiquement acceptable et un acide nucléique qui est un ARN antisens ou qui est pour l'expression dudit ARN antisens dans lesdites cellules proliférantes, où la composition pharmaceutique comprend ledit acide nucléique en une quantité efficace pour tuer ou inhiber la croissance des cellules proliférantes, ledit ARN antisens étant capable de s'hybrider avec un ARN transcrit dans lesdites cellules proliférantes pour ainsi former un ARN double brin ayant une longueur d'au moins 30 nucléotides,
où la composition pharmaceutique comprend en outre un interféron pour activer, et/ou augmenter les taux d'une protéine kinase dépendante de l'ARN double brin dans les cellules proliférantes.

22. Composition pharmaceutique pour traiter un trouble **caractérisé par** des cellules proliférantes exprimant une protéine kinase dépendante de l'ARN double brin, la composition pharmaceutique comprenant un véhicule physiologiquement acceptable et un acide nucléique qui est une molécule antisens ou qui code pour ladite molécule antisens, ladite molécule antisens étant capable de s'hybrider avec une séquence d'ARN contiguë qui englobe une région de délétion du Δ 2-7 EGFR de sorte à former un ARN double brin de 30-100 pb dans des cellules proliférantes exprimant ledit Δ 2-7 EGFR.

23. Composition pharmaceutique selon la revendication 22,
où lesdites cellules proliférantes expriment ladite kinase.

24. Composition pharmaceutique selon la revendication 21,
où la composition pharmaceutique comprend en outre un interféron pour activer, et/ou augmenter les taux d'une protéine kinase dépendante de l'ARN double brin dans lesdites cellules proliférantes.

25. Composition pharmaceutique selon la revendication 23,
où la composition pharmaceutique comprend en outre un interféron pour activer, et/ou augmenter les taux de ladite kinase dans lesdites cellules proliférantes.

26. Composition pharmaceutique selon la revendication 22
ou 23, où ledit ARN antisens est sélectionné dans le groupe consistant en SEQ ID NO: 1, SEQ ID NO: 3, SEQ ID NO: 5, SEQ ID NO: 7, un ARN codé par SEQ ID NO: 2, un ARN codé par SEQ ID NO: 4, un ARN codé par SEQ ID NO: 9, un ARN codé par SEQ ID NO: 11 et un ARN codé par SEQ ID NO: 13.

27. Composition comprenant un ARN antisens, ledit ARN antisens étant sélectionné dans le groupe consistant en SEQ ID NO: 1, SEQ ID NO: 3, SEQ ID NO: 5, SEQ ID NO: 7, un ARN codé par SEQ ID NO: 2, un ARN codé par SEQ ID NO: 4, un ARN codé par SEQ ID NO: 9, un ARN codé par SEQ ID NO: 11 et un ARN codé par SEQ ID NO: 13.

28. Acide nucléique comprenant une séquence de nucléotides codant pour un ARN antisens, ledit ARN antisens étant sélectionné dans le groupe consistant en SEQ ID NO: 1, SEQ ID NO: 3, SEQ ID NO: 5, SEQ ID NO: 7, un ARN codé par SEQ ID NO: 2, un ARN codé par SEQ ID NO: 4, un ARN codé par SEQ ID NO: 9, un ARN codé par SEQ ID NO: 11 et un ARN codé par SEQ ID NO: 13.

29. Vecteur comprenant une séquence de nucléotides codant pour un ARN antisens, ledit ARN antisens étant sélectionné dans le groupe consistant en SEQ ID NO: 1, SEQ ID NO: 3, SEQ ID NO: 5, SEQ ID NO: 7, un ARN codé par SEQ ID NO: 2, un ARN codé par SEQ ID NO: 4, un ARN codé par SEQ ID NO: 9, un ARN codé par SEQ ID NO: 11 et un ARN codé par SEQ ID NO: 13.

30. Vecteur selon la revendication 29, où ledit vecteur est un vecteur rétroviral.

31. Vecteur selon la revendication 30, où ledit vecteur rétroviral est un lentivirus.

32. Cellule transformée avec un vecteur selon la revendication 30.

33. Cellule selon la revendication 32, où la cellule est stablement transfectée.
